(19) Europäisches Patentamt
European Patent Office
Office européen des brevets

(11) Veröffentlichungsnummer: **0 169 408**
**B1**

(12) # EUROPÄISCHE PATENTSCHRIFT

(45) Veröffentlichungstag der Patentschrift: **31.05.89**

(21) Anmeldenummer: **85108070.5**

(22) Anmeldetag: **28.06.85**

(51) Int. Cl.[4]: **C 07 D 233/60**, C 07 D 401/12, C 07 D 405/12, C 07 D 409/12, A 61 K 31/435 // C07D405/10, C07F9/40

(54) **Neue Imidazolylverbindungen, Verfahren zu ihrer Herstellung und ihre Verwendung als Arzneimittel.**

(30) Priorität: **06.07.84 DE 3424944**

(43) Veröffentlichungstag der Anmeldung: **29.01.86 Patentblatt 86/5**

(45) Bekanntmachung des Hinweises auf die Patenterteilung: **31.05.89 Patentblatt 89/22**

(84) Benannte Vertragsstaaten: **AT BE CH DE FR GB IT LI LU NL SE**

(56) Entgegenhaltungen:
**EP-A-0 106 060**
**DE-A-2 923 815**

**J. Med. Chem., 1981, 24, 1139-1148**

(73) Patentinhaber: **HOECHST AKTIENGESELLSCHAFT, Postfach 80 03 20, D-6230 Frankfurt am Main 80 (DE)**

(72) Erfinder: **Wess, Günther, Dr., Hainstrasse 35, D-6455 Erlensee (DE)**
Erfinder: **Bartmann, Wilhelm, Dr., Am Dachsbau 5, D-6232 Bad Soden am Taunus (DE)**
Erfinder: **Beck, Gerhard, Dr., Gustav- Freytag- Strasse 24, D-6000 Frankfurt am Main 1 (DE)**
Erfinder: **Lau, Hans- Hermann, Dr., Kastanienhain 29, D-6232 Bad Soden am Taunus (DE)**

**EP 0 169 408 B1**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER. STOCKHOLM 1989

**Beschreibung**

Imidazol und seine in Position 1 substituierten Derivate sind Hemmer der Thromboxan-Synthetase (H.-H. Tai, Biochem. and Biophys. Res. Comm. 80, 236 (1978)).

Das Enzym Thromboxan-Synthetase katalysiert innerhalb des Arachidonsäuremetabolismus die Umwandlung der Prostaglandinendoperoxide ($PGH_2$ bzw. $PGG_2$) in Thromboxan $A_2$ ($TXA_2$). $TXA_2$ ist biologisch hoch aktiv: es induziert die Aggregation der Blutplättchen und wirkt außerdem auf die glatte Muskulatur stark konstriktiv. Es spielt eine wesentliche Rolle bei der Haemostase, bei pathologischen Situationen mit erhöhter Tendenz zu Gefäßkrämpfen und/oder Thrombose. Ferner wirkt $TXA_2$ in vitro und in vivo stark kontrahierend auf die Bronchialmuskulatur (B. Samuelsson, Angew. Chem 95, 854 (1983).

Die neuen 1-Imidazolylmethylstyrole, die in der vorliegenden Erfindung beschrieben werden zeichnen sich durch eine spezifische Hemmwirkung auf die Thromboxan-Synthetase aus. Diese Wirkung ist überraschend, da die neuen Verbindungen im Gegensatz zu den Verbindungen entsprechend der Deutschen Offenlegungsschrift 2 923 815 am Phenylrest unpolare Gruppen als terminalen Substituenten tragen. Entsprechend dem Stand der Technik ist die Bedeutung dieser terminalen Gruppe wesentlich für die Wirksamkeit der Verbindungen; polare Gruppen, wie z. B. die COOH-Gruppe, führen zu gut wirksamen Verbindungen, während bei weniger polaren Gruppen ein Wirkungsabfall eintritt (vgl. z. B. Kinji Izuka, et al. J. Med. Chem. 1981, Vol. 24, 1139 - 1148).

Die neuen Verbindungen eignen sich daher zur Prophylaxe oder Behandlung von Krankheiten mit gestörter (erhöhter) Thrombocytenaggregationsneigung, sowie bei pathologisch erhöhten Thromboxanspiegeln, wie sie bei Ischämie, Angina pectoris, thromboembolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma und Apnoe, Entzündungskrankheiten sowie mikrovaskulären Komplikationen bei Diabetes mellitus gefunden werden. Die erfindungsgemäßen Verbindungen beeinflussen günstig Erkrankungen mit erhöhten Thromboxanspiegeln in verschiedenen Organen, beispielsweise im Bereich der Nieren oder im Magen-Darm Bereich bei Colitis oder bei "inflammatory bowel disease". Außerdem sind die Verbindungen geeignet, die Proliferation von Tumorzellen zu verlangsamen bzw. zu verhindern.

Gegenstand der vorliegenden Erfindung sind neue ortho-, meta- und parasubstituierte Imidazolylmethylstyrole der Formel I

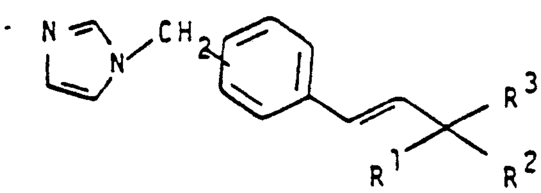

I

o, m, p

In der allgemeinen Formel I bedeuten:

$R^1$ und $R^2$ zusammen mit dem sie tragenden Kohlenstoffatom die Carbonylgruppe, oder $R^1$ Wasserstoffatom die darstellt oder

a) einen verzweigten oder unverzweigten aliphatischen Acylrest mit bis zu 10 C-Atomen,

b) den Benzylcarbonyl oder Benzoylrest, wobei der Phenylrest unsubstituiert oder 1- bis 3-fach substituiert ist mit Halogen oder $C_1$-$C_4$-Alkyl,

c) den Rest 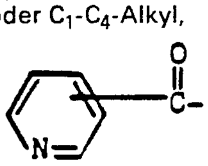

d) verweigtes oder unverzweigtes Alkyl mit 1 - 10 Atomen,

e) den Benzylrest, wobei der Phenylkern unsubstituiert oder 1-bis 3-fach substituiert ist mit Halogen oder $C_1$-$C_4$-Alkyl, oder

f) den Rest 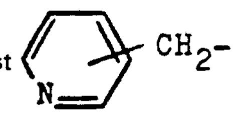 ,

$R^3$ einen Phenylrest, der im Kern 1- bis 3-fach substituiert sein kann mit Halogen, Trifluormethyl und/oder Alkyl oder Alkoxy mit je 1 - 4 C-Atomen, oder einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die

aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3 - 7 C-Atomen, einem unsubstituierten Phenyl-, α- oder β-Thienyl oder α- oder β-Furylrest oder einem Phenyl-, Thienyl oder Furylrest, welche ihrerseits im Kern 1- bis 3-fach substituiert sind mit Halogen, Trifluormethyl und/oder Alkyl oder Alkoxy mit 1 - 4 C-Atomen,

c) einem unsubstituierten Phenoxy-, α- oder β-Thienyloxyoder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen,

d) einem 1-Imidazolylrest.

Die Substituenten haben bevorzugt die folgende Bedeutung:

$R^1$ und $R^2$ zusammen mit dem sie tragenden Kohlenstoffatom die Carbonylgruppe, oder R "Wasserstoff und $R^2$ der Rest $-OR^4$, Wasserstoff darstellt oder

a) verzweigtes oder unverzweigtes Alkanoyl mit bis zu 6 C-Atomen,

b) den Benzylcarbonyl- oder Benzoylrest, wobei der Phenylkern unsubstituiert oder einfach substituiert ist mit Fluor, Chlor oder Methyl,

c) den Rest 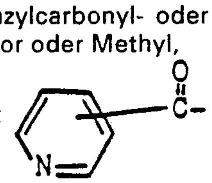

d) verzweigtes oder unverzweigtes Alkyl mit 1 - 6 C-Atomen,

e) den Benzylrest, wobei der Phenylkern unsubstituiert oder einfach substituiert ist mit Chlor, Fluor oder Methyl, oder

f) den Rest 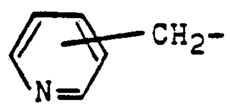

$R^3$ einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Cycloalkyl mit 3 - 7 C-Atomen, einem unsubstituierten Phenyl-, α- oder β-Thienyl oder α- oder β-Furylrest

c) einem unsubstituierten Phenoxy-, α- oder β-Thienyloxyoder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen,

d) einem 1-Imidazolylrest.

Besonders bevorzugt sind die folgenden Substituenten:

$R^1$ und $R^2$ zusammen mit dem sie tragenden Kohlenstoffatom die Carbonylgruppe, oder $R^1$ Wasserstoff und $R^2$ den Rest $-OR^4$ worin $R^4$ Wasserstoff, Benzyl, Alkyl, mit 1-6 C-Atomen oder den Rest 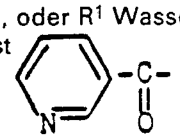

oder 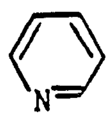 $-CH_2$-darstellt,

$R^3$ einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasser stoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen

b) Cycloalkyl mit 3 - 7 C-Atomen, einem unsubstituierten Phenyl-, α- oder β-Thienyl oder α- oder β-Furylrest oder einen Phenyl-, Thienyl- oder Furylrest,

c) einem unsubstituierten Phenoxy-, α- oder β-Thienyloxy- oder Cycloalkoxyrest mit 3 - 7 Kohlenstoffatomen

d) einem 1-Imidazolylrest

insbesondere die Reste:

n-Pentyl, 1,1-Dimethylpentyl, Cyclopentylmethyl, Cyclohexylmethyl, Methoxymethyl, Ethoxymethyl. Propoxymethyl, Butoxymethyl, Pentoxymethyl, Hexyloxymethyl, Heptyloxymethyl, Cyclopentyloxymethyl Cyclohexyloxymethyl, Dimethylpentoxymethyl, 1,1-Dimethyl-2-ethoxyethyl, Phenoxymethyl, 2-Ethoxyethyl, 2-Butoxyethyl, 3-Ethoxypropyl, 5-Methoxypentyl, (2-Ethoxy)ethoxymethyl, 3-Chlorphenoxymethyl, 1,1-Dimethyl-2-benzyloxyethyl, 3-Methoxycarbonylpropyl, 4-Methoxycarbonylbutyl, 5-Methoxycarbonylpentyl, 3-Carboxypropyl, 4-Carboxybutyl, 5-Carboxypentyl, 4-(3-Chlorbenzyloxy)phenyl, Phenylethoxymethyl, 3-Thienyloxymethyl, 2-Thienyloxymethyl, 2-(1-Imidazolyl)ethyl, Dimethyl-(1-imidazolyl).

Die Erfindung umfaßt ferner Säureadditionssalze mit anorganischen oder organischen Säuren z. B. Chlorwasserstoffsäure, Bromwasserstoffsäure, Schwefelsäure, Phosphorsäure oder Salpetersäure, Essigsäure, Propionsäure, Oxalsäure, Malonsäure, Glykolsäure, Bernsteinsäure, Maleinsäure, Fumarsäure, Apfelsäure, Weinsäure, Zitronensäure, Benzoesäure oder Zimtsäure.

Gegenstand der Erfindung ist ferner ein Verfahren zur Herstellung der Verbindungen der allgemeinen Formel I, dadurch gekennzeichnet, daß man

a) die entsprechenden ortho-, meta- und parasubstituierten 1-Imidazolylmethylbenzaldehyde der Formel II

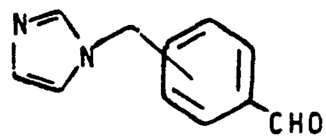

II

nach Horner-Emmons-Wittig mit einem Phosphonsäureester der allgemeinen Formel III

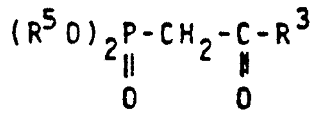

III

wobei $R^3$ die in der allgemeinen Formel I angegebene Bedeutung besitzt und $R^5$ $C_1$-$C_4$-Alkyl, bedeutet, zu den erfindungsgemäßen Verbindungen der allgemeinen Formel IV umsetzt,

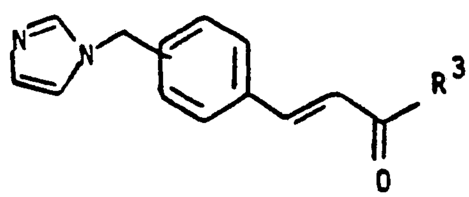

IV

wobei $R^3$ die zur Formel I angegebene Bedeutung hat,

b) gegebenenfalls die Enone der allgemeinen Formel IV mit einem Reduktionsmittel zu den erfindungsgemäßen Alkoholen der allgemeinen Formel V reduziert,

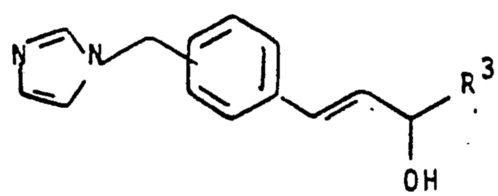

V

worin $R^3$ die zur Formel I gegebene Bedeutung hat,

c) gegebenenfalls die Alkohole der Formel V in Form ihrer Racemate oder als reine Enantionmere mit einem reaktionsfähigen Derivat einer Carbonsäure zu den erfindungsgemäßen Estern der allgemeinen Formel VI umsetzt,

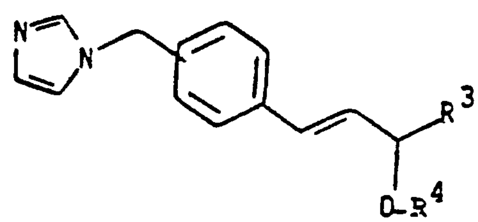

VI

worin $R^3$ die zur Formel I gegebene Bedeutung hat und $R^4$ einen verzweigten oder unverzweigten aliphatischen Acylrest mit bis zu 10 C-Atomen, oder den Benzylcarbonyl- oder Benzoylrest, worin der Phenylkern 1- bis 3-fach mit Halogen oder $C_1$-$C_4$-Alkyl substituiert sein kann, oder den Rest

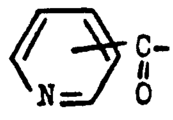

bedeutet, oder

d) gegebenenfalls die Alkohole der Formel V in Form ihrer Racemate oder als reine Enantionmere mit einem entsprechenden Halogenid, Tosylat oder Mesylat zu den erfindungsgemäßen Ethern der allgemeinen Formel VII umsetzt,

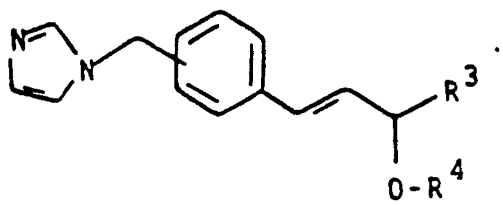

VII

worin $R^4$ $C_1$-$C_{10}$-Alkyl, Benzyl, worin der Phenylkern 1-bis 3-fach mit Halogen oder $C_1$-$C_6$-Alkyl substituiert sein kann, oder den Rest 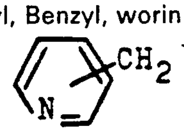 - darstellt.

Zur Herstellung der im erfindungsgemäßen Verfahren als Ausgangsmaterial verwendeten 1-Imidazolylmethylbenzaldehyde der Formel II setzt man die entsprechenden Chlormethylbenzaldehyde bzw. ihre Acetale mit Imidazol in Gegenwart einer Base um. Eine bevorzugte Ausführungsform besteht darin, daß man die Ethylenacetale der o-, m- und p-Chlormethylbenzaldehyde mit Imidazol in Toluol als Lösungsmittel unter Verwendung von Kaliumcarbonat als Base bei geeigneter Temperatur, vorzugsweise bei Rückflußtemperatur umsetzt.

Die Chlormethylbenzaldehyde können in Analogie zu bekannten Verfahren (z. B. R. Grice, L.N. Owen, J. Chem. Soc. 1963, 1947 oder I.W. Baker, I.A.L. Brieux, D.G. Saunders, J. Chem. Soc. 1956, 404) hergestellt werden.

Die o-, m-, p-(1-Imidazolylmethyl)benzaldehyde der Formel II werden nach Horner-Emmons-Wittig mit einem Phosphonsäureester der allgemeinen Formel III umgesetzt, wobei eine bevorzugte Ausführungsform darin besteht, daß man den Phosphonsäureester der Formel III mit dem Aldehyd der Formel II in Dimethoxyethan unter Verwendung von DBU (1,8-Diazabicyclo[5.4.0]undec-7-en) als Base bei Raumtemperatur bis Rückflußtemperatur 1 - 36 Stunden reagieren läßt.

Die Phosphonsäureester der Formel III können nach literaturbekannten Verfahren (siehe z. B. J. Am. Chem. Soc. 88, 5654 (1966)) hergestellt werden.

Die Alkohole der allgemeinen Formel V erhält man in Form ihrer Racemate, wenn man ein Enon der allgemeinen Formel IV mit einem komplexen Metallhydrid, vorzugsweise mit einem Alkaliboranat, vorzugsweise zwischen -10 °C und Raumtemperatur in Methanol, Ethanol oder Ethern wie DME, THF ggf. unter Wasserzusatz reduziert.

Die Acylierung der Alkohole der Formel V zu den Estern der Formel VI erfolgt in der allgemein üblichen Weise mit Acylhalogeniden (wie z. B. Nicotinsäurechlorid) oder mit Säureanhydriden in Gegenwart von Basen wie Pyridin, Triethylamin u. a.

Die Veretherung der Alkohole der Formel V zu den Ethern der Formel VII erfolgt in der allgemein üblichen Weise mit Halogeniden (wie z. B. Benzylhalogenid), Mesylaten oder Tosylaten in Gegenwart von Basen wie z. B. Natriumhydrid in geeigneten Lösungsmitteln wie z. B. DMF.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation oder Säulen-, Dünnschicht- oder Hochdruckflüssigkeitschromatographie.

Die Säureadditionssalze von Verbindungen der allgemeinen Formel I werden durch Zugabe der entsprechenden anorganischen oder organischen Säuren meistens in Form einer Lösung zu Lösungen der entsprechenden Imidazolverbindung der allgemeinen Formel I erhalten. Als Lösungsmittel dienen z. B. Wasser, Alkohole aber auch verschiedene Ether und Ester.

Außer den in den Beispielen beschriebenen Verbindungen lassen sich nach den erfindungsgemäßen Verfahren die folgenden Verbindungen herstellen:

E-4-Ethoxy-1-[4-(1-imidazolylmethyl)phenyl]but-1-en-3-ol
E-5-Ethoxy-1-[4-(1-imidazolylmethyl)phenyl]pent-1-en-3-ol
E-5-Butoxy-1-[4-(1-imidazolylmethyl)phenyl]pent-1-en-3-ol
E-6-Ethoxy-1-[4-(1-imidazolylmethyl)phenyl]hex-1-en-3-on

E-6-Ethoxy-1-[4-(1-imidazolylmethyl)phenyl]hex-1-en-3-ol
E-7-Methoxy-1-[4-(1-imidazolylmethyl)phenyl]hept-1-en-3-on
E-7-Methoxy-1-[4-(1-imidazolylmethyl)phenyl]hept-1-en-3-ol
E-8-Methoxy-1-[4-(1-imidazolylmethyl)phenyl]oct-1-en-3-on
E-8-Methoxy-1-[4-(1-imidazolylmethyl)phenyl]oct-1-en-3-ol
E-5-(1-Imidazolyl)-1-[4-(1-imidazolylmethyl)phenyl]pent-1-en-3-ol
E-4,4-Dimethyl-4-(1-imidazolyl-1-[4-(1-imidazolylmethyl)phenyl]but-1-en-3-on
E-4,4-Dimethyl-4-(1-imidazolyl)-1-[4-(1-imidazolylmethyl)phenyl]but-1-en-3-ol
E-3-(3-Chlorphenyl)-1-[4-(1-imidazolylmethyl)phenyl]prop-1-en-3-on
E-3-(3-Chlorphenyl)-1-[4-(1-imidazolylmethyl)phenyl]prop-1-en-3-ol

sowie die Nicotinsäureester, Benzylether und [(3-Pyridyl)-methyl]ether der hier und in Tabelle 2 aufgeführten Alkohole.

Die verbindungen der Formel I zeichnen sich durch eine spezifische Hemmwirkung auf die Thromboxan-Synthetase aus und können daher als Arzneimittel zur Prophylaxe oder Behandlung von Krankheiten mit gestörter (erhöhter) Thrombocytenaggregationsneigung, sowie bei pathologisch erhöhten Thromboxanspiegeln, wie sie bei Ischämie, Angina pectoris, thrombo-embolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma und Apnoe, Entzündungskrankheiten sowie mikrovaskulären Komplikationen bei Diabeters mellitus gefunden werden. Die erfindungsgemäßen Verbindungen beeinflussen günstig Erkrankungen mit erhöhten Thromboxanspiegeln in verschiedenen Organen, beispielsweise im Ereich der Nieren oder im Magen-Darm-Bereich bei Colitis oder bei "inflammatory bowel disease". Außerdem sind die Verbindungen geeignet, die Proliferation von Tumorzellen zu verlangsamen bzw. zu verhindern. Die Verbindungen können in Tagesdosen von 0,01 mg/kg bis 10 mg/kg, bevorzugt 0,1 mg/kg bis 5 mg/kg, und in Einzeldosen von 0,01 mg/kg bis 7,5 mg/kg, bevorzugt 0,01 mg/kg bis 2,5 mg/kg verabreicht werden.

Arachidonsäuremetabolite sind an einer Vielzahl physiologischer und pathophysiologischer Prozesse beteiligt. Bei der Regulation des Tonus der Blutgefäße und der Blutplättchenaggregation spielen Prostacyclin ($PGI_2$) und Thromboxan $A_2$ ($TXA_2$) eine wesentliche Rolle. Prostacyclin, das bevorzugt in den Endothelzellen der Blutgefäße aus Prostaglandinoperoxid $H_2$ ($PGH_2$) entsteht, bewirkt eine Vasodilatation und verhindert die Aggregation der Blutplättchen. Die Umwandlung von Prostaglandinendoperoxid in Prostacyclin wird durch das Enzym Prostacyclin-Synthetase katalysiert. Thromboxan $A_2$ ist der physiologische Gegenspieler des Prostacyclins. Es entsteht vornehmlich in den Blutplättchen aus $PGH_2$. Das Enzym Thromboxan-Synthetase katalysiert diese Reaktion. $TXA_2$ bewirkt eine Aggregation der Blutplättchen und führt zu einer Vasokonstruktion. Sowit bekannt, ist Thromboxan der potenteste Vasokonstriktor im menschlichen Organismus (A.G. Herman, P.M. Vonhoutte, H. Denolin, A. Goossens, Cardiovascular Pharmacology of the Prostaglandins, Raven Press, New York 1982). Ungleichgewichte zwischen Prostacyclin und Thromboxan $A_2$ führen zu pathophysiologischen Situationen. Eine Verschiebung des Gleichgewichtes zugunsten des Thromboxans führt daher zu einer Blättchenaggregation und Gefäßspasmen, sowie einer erhöhten Anfälligkeit gegenüber Atherothrombose (Lancet 1977, 479; Science 1976, 1135; Amer. J. Cardiology 41, 787 (1978); Lancet 1977, 1216;). Bei der experimentellen Atherosklerose ist die $PGI_2$-Bildung unterdrückt und die $TXA_2$-Bildung erhöht (Prostaglandins 14, 1025 und 1035 (1977)). Daher wird Thromboxan $A_2$ mit verschiedenen Anginen, myocardialer Infarktbildung, plötzlichem Herztod und Schlaganfällen in Verbindung gebracht (Thromb. Haemostasis 38, 132 (1977); Platelets, Prostaglandins and Cardiovascular System, Florenz, Februar 1984).

Ein anderes Gebiet, auf welchem ein Ungleichgewicht von $PGI_2$ / $TXA_2$ als ein beitragender Faktor angesehen wird, ist dasjenige von Migräne. Migränekopfschmerz ist mit Veränderungen der intra- und extracerebralen Blutströmung verbunden, insbesondere mit einervor dem Auftreten des Kopschmerzes erfolgenden Reduzierung der cerebralen Blutströmung und anschließenden Dilatation in beiden Gefäßbereichen während der Kopfschmerzphase. Blutplättchen von Migränepatienten besitzen eine größere Neigung zur Aggregation als diejenigen von normalen Individuen (J. Clin. Pathol. 24, 250 (1971); J. Headache, 17, 101 (1977); Lancet 1978, 501).

Bei Patienten mit Diabetes mellitus wird ein Ungleichgewicht zwischen Prostacyclin und Thromboxan $A_2$ als verantwortlich für die microvasculären Komplikationen angegesehen. Plättchen von Diabetespatienen bilden erhöhte Mengen an $TXB_2$ und Malondialdehyd, siche Symposium "Diabetes and Thrombosis-Implications for Therapy", Leeds, Großbritannien (April 1979). Weiterhin wurde gezeigt, daß bei Ratten mit experimentell erzeugter Diabetes die vaskuläre Prostacyclinbildung gehemmt wird und die $TXA_2$-Synthese aus den Plättchen erhöht ist, siehe IV. International Prostaglandin Conference, Washington, D.C. (Mai 1979).

Nichtsteroidale Antiinflammatorika hemmen die Cyclooxygenase, die die Umwandlung von Arachidonsäure in $PGH_2$ über $PGG_2$ katalysiert. Sie greifen daher in die Biosynthese sowohl des Thromboxans als aber auch in die Biosyntheses des Prostacyclins ein. Wertvoller wäre daher eine Verbindung, die spezifisch die Bildung von $TXA_2$ durch Blockade der $TXA_2$-Synthetase hemmt und den Prostacyclinweg unbeeinflußt läßt.

Die Verbindungen der Formel I eignen sich daher zur Vorbeugung oder Behandlung der oben aufgeführten Erkrankungen, die auf eine Hemmung der Thromboxansynthetase ansprechen.

Die Verbindungen der Formel I werden in verschiedenen Dosierungsformen verabreicht, z. B. oral in Form von Tabletten, Kapseln oder Flüssigkeiten, rektal in Form von Suppositorien, parenteral, subkutan oder

intramuskulär, wobei intravenöse Verabreichung in Notsituationen bevorzugt wird.

Die erfindungsgemäßen Verbindungen der Formel I können als freie Basen oder in Form ihrer physiologisch unbedenklichen anorganischen oder organischen Säureadditionssalze zu Anwendung kommen. Die freien Basen und Säureadditionssalze können in Form ihrer wässrigen Lösungen oder Suspensionen oder auch gelöst oder suspendiert in pharmakologisch unbedenklichen organischen Lösungsmitteln wie ein- oder mehrwertigen Alkoholen wie z. B. Ethanol, Ethylenglykol oder Glycerin, in Triacetin, in Alkohol-Acetaldehyddiacetalgemischen, Ölen wie z. B. Sonnenblumenöl oder Lebertran, Ethern wie z. B. Diethylenglykoldimethylether oder auch Polyethern wie z. B. Polyethylenglykol oder auch in gegenwart anderer pharmakologisch unbedenklicher Polymerträger wie z. B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen Suppositorien oder Ärosole in Frage kommen.

**Beispiel 1a**

4-(1-Imidazolylmethyl)benzaldehydethylenacetal

54 g (0,27 Mol) 4-Chlormethylbenzaldehydethylenacetal, 20 g (0,29 Mol) Imidazol und 70 g (0,5 Mol) Kaliumcarbonat wurden in 800 ml Toluol 2 h unter Rühren am Rückfluß erhitzt. Nach dem Erkalten setzte man ca. 500 ml Wasser zu und rührte 15 min. intensiv bei Raumtemp.. Die Phasen wurden getrennt und die wäßrige 2x mit Toluol extrahiert. Trocknen der vereinigten organischen Phasen über Magnesiumsulfat und Abziehen des Lösungsmittels im Vakuum ergab ein gelbliches Öl, das über eine kuze Kieselgelsäule chromatographiert wurde (Chloroform / Methanol = 10 : 1) Ausbeute: 44.3 g (0,192 Mol, 71 %) als gelbliches Öl.

$^1$H-NMR ($CDCl_3$, 60 MHz) δ; 4,0 (m, 4H, $-OCH_2CH_2O-$), 5,1 (s, 2H, $-CH_2-$), 5,8 (s, 1H, Methin-H), 6,8 - 7,7 (m, 7H, arom. H)

1b: In Analogie zu 1a wird 2-(1-Imidazolylmethyl)benzaldehydethylenacetal erhalten.

$^1$H-NMR ($CDCl_3$, 60 MHz) δ = 4,0 (m, 4H, $-OCH_2CH_2O-$), 5,2 (s, 2H, $-CH_2-$), 5,7 (s, 1H, Methin-H), 6,7 - 7,9 (m, 7H, arom. H).

1c: In Analogie zu 1a wird 3-(1-Imidazolylmethyl)benzaldehydethylenacetal erhalten.

$^1$H-NMR ($CDCl_3$, 60 MHz) δ = 4,1 (m, 4H, $-OCH_2CH_2O-$), 5,0 (s, 2H, $-CH_2-$), 5,8 (s, 1H, Methin-H), 6,7 - 7,7 (m, 7H, arom H).

**Beispiel 2a**

Darstellung von 4-(1-Imidazolylmethyl)benzaldehyd

44,3 g (0,192 Mol) 4-(1-Imidazolylmethyl)benzaldehydethylenacetal wurden in 450 ml 1n wäßriger Salzsäure 3,5 h bei Raumtemp. gerührt. Man neutralisierte mit gesättigter wäßriger Natriumhydrogencarbonatlösung, sättigte mit Natriumchlorid und extrahierte dreimal mit Toluol. Die vereinigten organischen Phasen wurden über Magnesiumsulfat getrocknet. Abziehen des Lösungsmittels im Vakuum ergab 27,1 g (0,146 Mol, 76 %) Aldehyd als gelbliches Öl.

IR (Film): 1700 $cm^{-1}$ (s, Carbonyl)

δ = 5,2 (s, 2H, $-CH_2-$), 6,8 - 7,9 (m, 7H, arom. H), 9,9 (s, 1H, -CHO)

2b: In Analogie zu 2a wird 2-(1-Imidazolylmethyl)benz-aldehyd erhalten:

$^1$H-NMR ($CDCl_3$, $CDCl_3$) δ = 5,55 (s, 2H, $-CH_2-$), 6,8 - 7,9 (m, 7H, arom. H), 9,9 (s, 1H, -CHO)

2c: In Analogie zu 2a wird 3-(1-Imidazolylmethyl)benzaldehyd erhalten.

$^1$H-NMR ($CDCl_3$, 60 MHz) δ = 5,3 (s, 2H, $-CH_2-$), 6,7 - 7,9 (m, 7H, arom. H), 9,8 (s, 1H, -CHO)

**Beispiel 3**

2-Oxo-3-pentyloxypropanphosphonsäuredimethylester

Unter Argonatmosphäre wurden in 200 ml trockenem Tetrahydrofuran 25,6 g (0,2 Mol) Methanphosphonsäuredimethylester vorgelegt. In zwei Tropftrichtern wurden jeweils 250 ml 1,6 molare hexanische Lithiumbutyllösung (0,4 Mol) und 100 ml einer Lösung von 32,04 g (0,2 Mol) 2-Pentyloxyessigsäuremethylester in Tetrahydrofuran vorgelegt. Bei -70°C bis -65°C wurden dann 125 ml der Lithiumbutyllösung und gleich danach 50 ml der Esterlösung zugetropft. Man rührte 1 h bei -70°C und tropfte anschließend weitere 63 ml der Lithiumbutyllösung und 25 ml der Esterlösung bei -70°C zu und ließ 1h bei dieser Temperatur rühren. Schließlich wurden die restlichen 62 ml der Lithiumbutyllösung und 25 ml der Esterlösung bei der gleichen Temp. zugetropft und 2 h gerührt. Man ließ über Nacht in Trockeneis stehen Zur Aufarbeitung wurde die Lösung bei 0 - 5°C mit ca 160 ml 2 n wäßriger Salzsäure auf pH 5 eingestellt und anschließend am Rotationsverdampfer das THF abgezogen. Der Rückstand wurde mit gesättigter wäßriger Natriumchloridlösung aufgenommen und viermal mit Essigester extrahiert. Trocknen der vereinigten

Essigesterphasen über Magnesiumsulfat, Abziehen des Lösungsmittels im Vakuum und fraktionierte Destillation ergab 37,0 g (0,147 Mol, 73 %) Phosphonat als farblose Flüssigkeit, Sdp. 130 - 132° C / 0.5 Torr.

IR (Film): 1730 $cm^{-1}$ (s, Carbonyl)

$^1$H-NMR (60 MHz, $CDCl_3$) $\delta$ = 0,8 - 1,8 (m, 9H, $-(CH_2)_3CH_3$), 3,16 (d, J=22Hz, 2H, P-$CH_2$-), 3,47 (t, J = 6Hz, 2H,-$OCH_2$-), 3,75 (d, J = 11Hz, 6H, -$OCH_3$), 4,1 (s, 2H, -$CH_2$-)

In Analogie zu Beispiel 3 werden die Phosphonate der Formel III erhalten. Die als Ausgangsmaterial verwendeten Ester sind weitgehend literaturbekannt oder werden in Analogie zu bekannnten Verfahren hergestellt.

**Beispiel 4a**

E-4-Pentyloxy-1-[4-(1-imidazolylmethyl)phenyl]but-1-en-3-on

2 g (7,93 mmol) 2-Oxo-3-pentyloxypropanphosphonsäuredimethylester, 1,7 g (9,13 mmol) 4-(1-Imidazolylmethyl) benzaldehyd und 1,33 g (8,74 mmol) DBU wurden in 150 ml Dimethoxyethan 31 h bei Raumtemperatur gerührt. Man filtrierte durch Kieselgel und zog das Lösungsmittel im Vakuum ab. Chromatographie auf Kieselgel (Chloroform / Methanol / 25 proz. wäßr. $NH_3$ = 20/1/0,2) ergab 1,5 g (4,80 mmol, 60 %) als gelbliches Öl, das im Kühlschrank erstarrt, Schmp. 35 - 36° C.

Rf = 0,5

IR (Film): 1690 $cm^{-1}$ (s), 1610 (s)

$^1$H-NMR ($CDCl_3$, 60 MHz) $\delta$ = 0,7 - 2,0 (m, 9H, Alkyl-H), 3,5 (t, 2H, -$OCH_2$-), 4,2 (s, 2H,-$OCCH_2O$-), 5,2 (s, 2H, N-$CH_2$-Ar), 6,7 - 8,0 (m, 9H, arom-H und vinyl-H).

In Analogie zu Beispiel 4a werden weitere u. a. die in Tab. 1 angeführten Beispiel 4b - 4v erhalten.

Tabelle 1

| Beispiel | 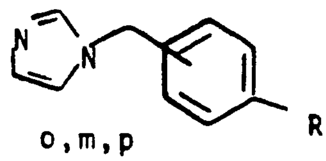 o,m,p R | charakteristische $^1$H-NMR-Signale ($CDCl_3$; 60 MHz), $\delta$ = | Rf. Kieselgel |
|---|---|---|---|
| 4b | 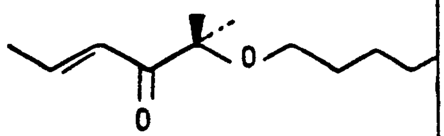 para | 0,58 - 2,0 (m, 15H, Methyl und -$(CH_2)_3CH_3$) | 0,24 $CHCl_3/CH_3OH/NH_4OH$ = 20:0,5:0,1 |
| 4c | 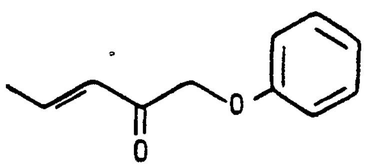 para | 4,73 (s, 2H, -$CH_2O$-) | 0,21 $CHCl_3/CH_3OH/NH_4OH$ = 20:0,4:0,1 |
| 4d | 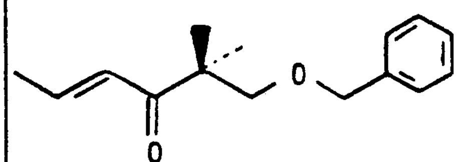 | 1,2(s,6H,$CH_3$) 3,5(s,2H,$CH_2O$) 4,5(s,2H,$CH_2OC_6H_5$) 5,1(s,2H,N-$CH_2$) 6,9-7,6 (m,14H, aromat. Prot.) | 0,46 $CH_2Cl_2$/MeOH = 20 : 1 |
| 4e | 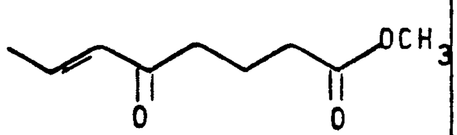 para | 1,9-2,9(m,6H, $CH_2$) 3,6(s,3H,$OCH_3$) 5,1(s,2H,N-$CH_2$) 6,5-7,7(m,9H, aromat. Prot.) | 0,36 $CH_2Cl_2$/MeOH = 20 : 1 |

| | | | |
|---|---|---|---|
| 4f | 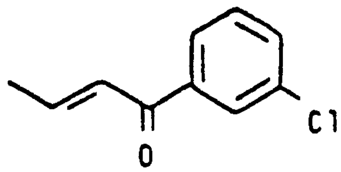 | 5,2 (s,2H,$CH_2$) 6,9-8,0 (m,13H, aromat. und olefin. Prot.) | 0,2 Essigester / MeOH = 8:1 |
| 4g | 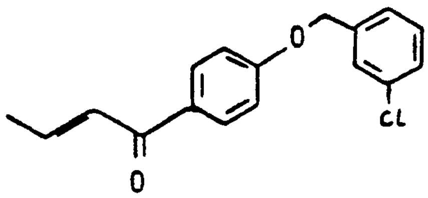 para | 5.1(s,2H, $CH_2OC_6H_4Cl$) 5.12(s,2H,NCH2) 6,9-8,1 (m,17H, olefinische u. aromat. Prot.) | 0,44 $CHCl_3$ / MeOH = 9:1 |
| 4h | 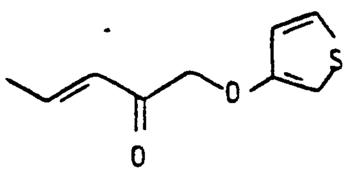 para | 4,8(s,2H, $CH_2O$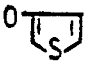) 5,15(s,2H,$CH_2N$) 6,15-6,25 (m, 1H Thiophen) 6,8-7,9(m,11H,Olefin +aromat. Prot.) | 0,22 $CHCl_3$/MeOH = 9:1 |
| 4i | 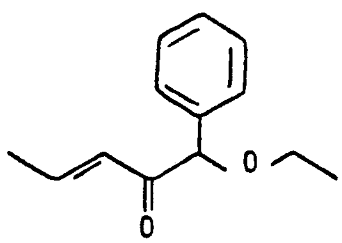 para | 1,2(t,3H,$OC_2H_5$) 3,5(q,2H,$OC_2H_5$) 5,1(s,2H,$CH_2H$) 6,9-8,0(m,14H olefin. + aromat. Prot.) | 0,26 $CHCl_3$/MeOH = 9:1 |
| 4j | 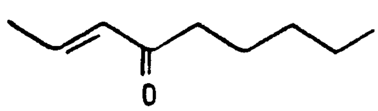 para | 0,6-2,1(m,9H, $(CH_2)_3CH_3$), 2,67 (t,2H, $-COCH_2-$) | 0,16 Essigester / $CH_3OH$ = 20:1 |

| | | | |
|---|---|---|---|
| 4k | 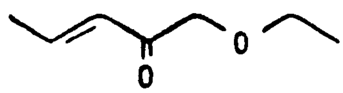 para | 1,33(t,3H,$-CH_3$) 3.63(q,2H,$-CH_2$) 4,2(s,2H,$-CH_2-$) | 0,19 Essigester / $CH_3OH$ = 20:1 |
| 4l | 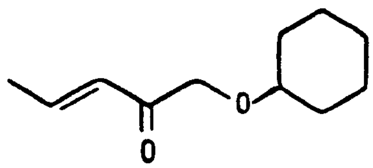 para | 1,0-2,2(m,10H, Methylen-H), 3,33 (m,1H, Methin-H), 4,23 (s,2H,$-OCH_2-$) | 0,14 Essigester / $CH_3OH$ = 20:1 |
| 4m | 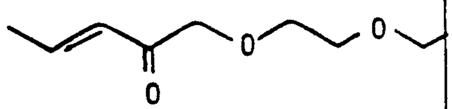 para | 1,23(t,3H,$-CH_3$) 3,33-3,66(m,6H, $-CH_2-$), 4,33(s, 2H,$-COCH_2O-$ | 0,14 Essigester / $CH_3OH$ = 20:1 |
| 4n | 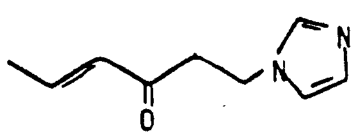 para | 3,1(t,2H,$CH_2CO$) 4,25(t,2H,$NCH_2$) 5,1(s,2H,$NCH_2$) 6,5-7,7(m,9H, olefin.+aromat. Prot.) | 0,17 Essigester / $CH_3OH$ = 2:1 |
| 4o | 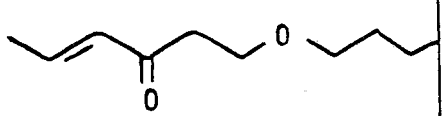 para | 0,8-1,6(m,7H, $C_3H_7$) 2,95(t,2H,$CH_2CO$) 3,2-3,95(t+q,4H, $2xCH_2O$) 5,1(s,$NCH_2$,2H) 6,5-7,8(m,9H, olefin + aromat. | 0,31 Essigester / $CH_3OH$ = 4:1 Prot) |

| | | | |
|---|---|---|---|
| 4p | 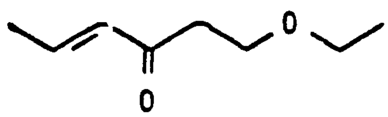 para | 1,15(t,3H,$OC_2H_5$) 2,95(t,2H,$CH_2CO$) 3,25-3,9(t+q,4H, 2x$CH_2O$) 5,1(s,2H,$NCH_2$) 6,5-7,9(m,9H, olefin + aromat. Prot.) | 0,45 Essigester / $CH_3OH$ = 4:1 |
| 4q | 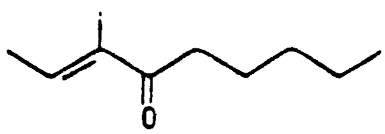 para | 0,9-1,9(m,9H, $CH_2$) 2,0(d,3H,$CH_3$) 2,8(t,2H,$CH_2CO$) 5,1(s,2H,N-$CH_2$) 6,9-7,7(m,9H, aromat. Prot.) | 0,20 $CH_2CL_2$/MeOH = 20:1 |
| 4r | 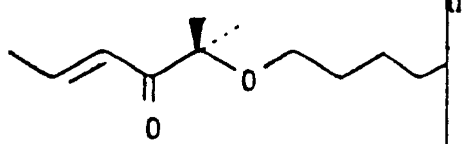 ortho | 0,6-2,1 (m,15H,Methyl-H und-$(CH_2)_3CH_3$) | 0,25 Essigester / $CH_3OH$ = 20:1 |
| 4s | 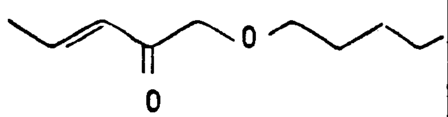 ortho | 0,8-2,1(m,9H, Alkyl-H) 3,5(t,2H,-$OCH_2$-) 4,2(s,2H, -$OCCH_2O$-) | 0,55 $CHCl_3$/$CH_3OH$/ 25 proz. wäßr. $NH_3$ = 20:1:0,2 |
| 4t | 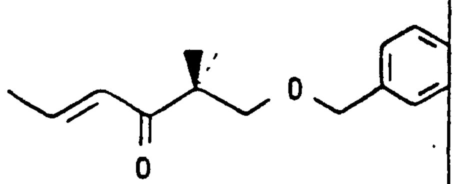 ortho | 1,2(s,6H,-$CH_3$), 3,5(s,2H,$CH_2O$), 4,5(s,2H, $CH_2OC_6H_5$) | 0,5 $CH_2Cl_2$/ $CH_3OH$ = 20:1 |

| | | | |
|---|---|---|---|
| 4u | 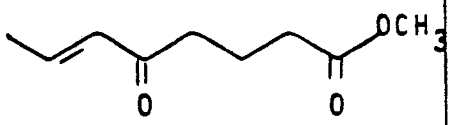 meta | 1,8-2,8(m,6H, $CH_2$), 3,6(s,3H, $OCH_3$) | 0,45 $CH_2Cl_2$/$CH_3OH$ = 20:1 |
| 4v | 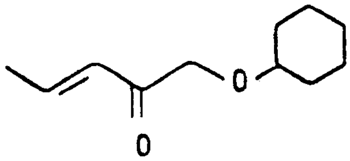 meta | 1,0-2,2(m,10H, Methylen-H), 3,3(m,1H, Methin-H), 4,2 (s,2H,-$OCH_2$-) | 0,25 Essigester/$CH_3OH$ = 20:1 |

**Beispiel 5a**

E-4-Pentyloxy-1-[4-(1-imidazolylmethyl)phenyl]but-1-en-3-ol

500 mg (1,60 mmol) Enon 4a wurden in 10 ml Methanol unter Eiskühlung mit 61 mg (1,61 mmol) Natriumborhydrid versetzt. Man rührte 1 h unter Eiskühlung, engte das Reaktionsgemisch im Vakuum ein, versetzte den Rückstand mit Wasser und stellte mit 1 N Salzsäure auf pH 7 ein. Die wäßrige Lösung wurde mit Natriumchlorid gesättigt und viermal mit Essigester extrahiert. Waschen der vereinigten organischen Phasen mit gesättigter Natriumchloridlösung und Trocknen über Magnesiumsulfat ergab nach Abziehen des Lösungsmittels im Vakuum 400 mg (1,27 mmol, 79 %) eines farblosen Öls.

Rf: 0,30 ($CHCl_3$/Methanol / 25 proz. wäßr. $NH_3$ = 20/1/0,2)

IR. (Film) 3250 $cm^{-1}$ (s)

$^1$H-NMR ($CDCl_3$, 60 MHz) $\delta$ = 0,6 - 2,0 (m, 9H, Alkyl-H), 2,7 (breit, 1H, OH), 3,3 - 3,8 (m, 4H,-$CH_2OCH_2$-), 4,2 - 4,7 (m, 1H, Methin-H), 5,1 (s, 2H, N-$CH_2$-Ar), 6,0 - 7,6 (m, 9H, arom-H und vinyl-H).

In Analogie zu Beispiel 5a werden weitere u. a. die in Tab. 2 aufgeführten Beispiel 5b - 5j erhalten.

Tabelle 2

| Beispiel | 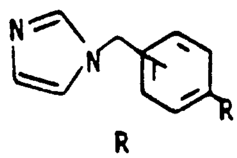 | charakteristische $^1H$-NMR-Signale ($CDCl_3$, 60 MHz), $\delta$ = | Rf Kieselgel |
|---|---|---|---|
| 5b | 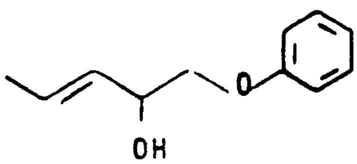 para | 4,0 (m,2H, $-CH_2O-$), 4,67 (m,1H, Methin-H), | 0,20 $CHCl_3/CH_3OH$ = 9:1 |
| 5c | 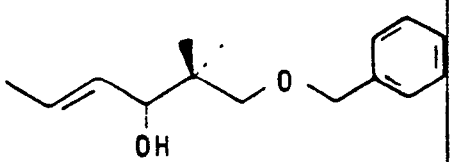 para | 0,95(d,6H,$CH_3$) 3,4(d,2H,$CH_2O$) 4,5(s,2H,$CH_2OC_6H_5$) 5,05(s,2H,$N-CH_2$) 6,0-7,6(m,14H, aromat. u. olefin. Prot.) | 0,28 $CH_2Cl_2$/ MeOH = 20:1 |

| | | | |
|---|---|---|---|
| 5d | 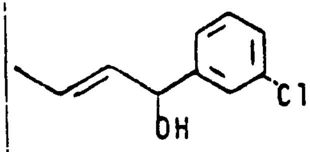 para | 5,1(s,2H,$CH_2$), 5,35(d,1H,CH-O), 6,1-6,45(dd,1H, =C<u>H</u>-CHO) 6,7(d,1H,=CH-Ph) 6,9-7,6(m,11H, aromat. H) | 0,29 Essigester/ $CH_3OH$ = 8:1 |
| 5e | 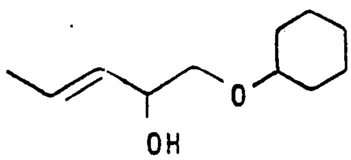 para | 1,0-2,3(m,10H, Methylen-H), 4,5(m,1H, Methin-H) | 0,32 $CHCl_3/CH_3OH$ = 9:1 |
| 5f | 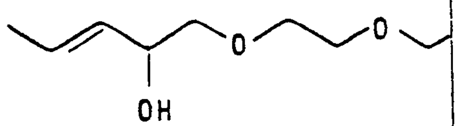 para | 1,23(t,3H,$CH_3$), 3,33-3,91(m,8H, $-CH_2-$), 4,5(m, 1H, Methin-H) | 0,42 $CHCl_3/CH_3OH$ = 9:1 |
| 5g | 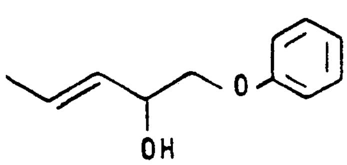 ortho | 4,0(m,2H,$-CH_2O-$) 4,68(m,1H, Methin-H) | 0,25 $CHCl_3/CH_3OH$ = 9:1 |

| | | | |
|---|---|---|---|
| 5h | 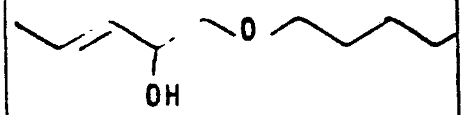 ortho | 0,7-2,0(m,9H, Alkyl-H), 3,3-3,8(m,4H, $-CH_2OCH_2-$) 4,3- 4,6(m,1H Methin-H) | 0,35 $CHCl_3/CH_3OH/$ 25 proz. wäßr. $NH_3$ = 20:1:0,2 |
| 5i | 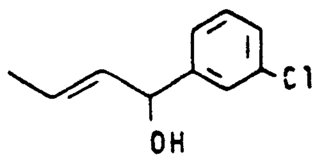 meta | 5,4(d,1H, Methin-H), 6,9-7,6(m,11H, arom. H) | 0,3 Essigester/ $CH_3OH$ = 9:1 |
| 5j | 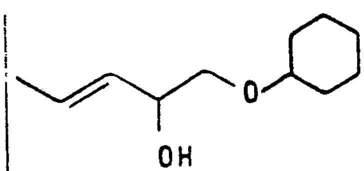 meta | 1,0-2,3(m,10H, Methylen-H), 4,5(m,1H, Methin-H) | 0,35 $CHCl_3/CH_3OH$ = 9:1 |

**Beispiel 6a**

E-4-Pentyloxy-3-(3-pyridylcarbonyloxy)-1-[4-(1-imidazolylmethyl-1-phenyl]but-1-en

400 mg (1,3 mmol) E-4-Pentyloxy-1-[4-(1-imidazolylmethyl)-phenyl]but-1-en-3-ol (5a) wurden in 10 ml trocknem Pyridin gelöst und mit 409 mg (1,5 mmol) 3-Pyridincarbonsäurechlorid-hydrochlorid versetzt. Man ließ 8 h bei Raumtemp. rühren, zog das Lösungsmittel im Vakuum weitgehend ab und verteilte den Rückstand zwischen gesättigter wäßriger Natriumhydrogencarbonatlösung und Methylenchlorid. Die wäßrige Phase wurde zweimal mit Methylenchlorid extrahiert. Trocknen der vereinigten organischen Phasen mit Magnesiumsulfat, Abziehen des Lösungmittels im Vakuum ergab nach Chromatographie auf Kieselgel (Essigester / Methanol = 10 : 1, Rf = 0,20) 380 mg (0,91 mmol, 70 %) Ester 6a als farbloses Öl.

IR(Film): 1720 $cm^{-1}$ (s, Estercarbonyl)

$^1H$-NMR (60 MHz, $CDCl_3$) $\delta$ = 0,70 - 1,8 (m, 9H, Alkyl-H), 3,3 - 3,8 (m, 4H, $-CH_2OCH_2-$), 5,1 (s, 2H, $N-CH_2$), 5,8 - 9,4 (m, 14H, Methin-H, aromatische H und olefinsiche H)

In Analogie zu Beispiel 6a wurden weitere u. a. die in Tabelle 3 aufgeführten Beispiele 6b - 6e erhalten

Tabelle 3

| Beispiel | 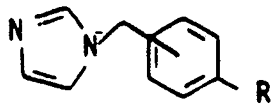 R = | charkateristische $^1$H-NMR-Signale, δ = | Rf Kieselgel |
|---|---|---|---|
| 6b | 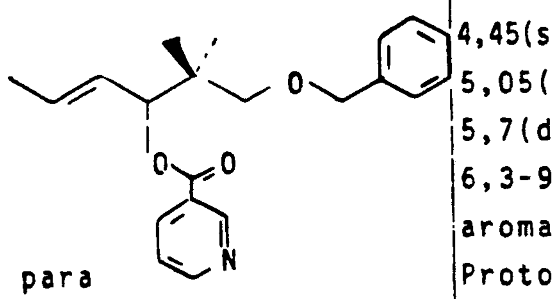 para | 1,1(d,6H,$CH_3$), 3,3(s,2H,$OCH_2$), 4,45(s,2H,$OCH_2C_6H_5$) 5,05(s,2H,N-$CH_2$) 5,7(d,1H, 6,3-9,3(m,18H, aromat + olefin. Protonen) | 0,32 $CH_2Cl_2$ / $CH_3OH$ = 20:1 |
| 6c | 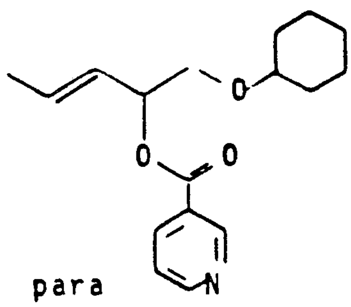 para | 1,0-2,2(m,10H, Cyclohexan-methylen-H), 3,78(d,2H, $CH_2O$-) 6,0-9,3 (m,13H, olefin. u. aromat. H) | 0,19 Essigester/ $CH_3OH$ = 20:1 |
| 6d | 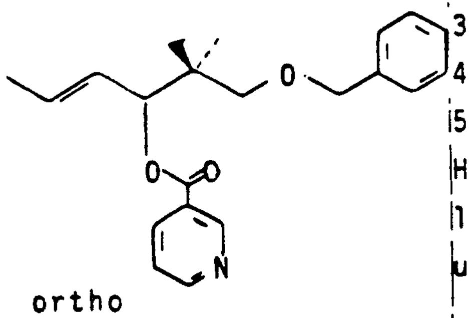 ortho | 1,1(d,6H,-$CH_3$), 3,3(s,2H,$OCH_2$), 4,5(s,2H,$OCH_2$Ph), 5,6(d,1H,Methin-H), 6,4-9,2 (m, 18H, olefin. u. aromat. H) | 0,35 $CH_2Cl_2$ / $CH_3OH$ = 20:1 |
| 6e | 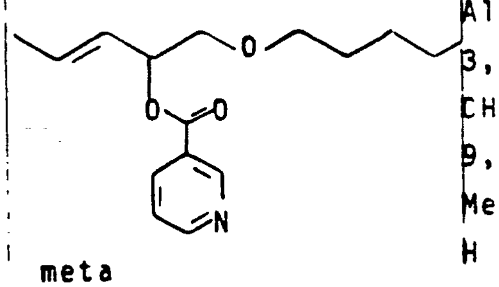 meta | 0,8-1,8(m,9H, Alkyl-H), 3,3-3,8 (m,4H,-$CH_2OCH_2$-), 5,7-9,4 (m,14H, Methin-H, olefin. H und aromat. H) | 0,2 Essigester/ $CH_3OH$ = 10:1 |

**Beispiel 7a**

E-4-Pentyloxy-1-[4-(1-imidazolylmethyl)phenyl]but-1-en-3-yl-benzylether

70 mg (1,6 mmol) 55 proz. NaH-Dispersion wurden in 3 ml trockenem Dimethylformamid vorgelegt. Bei Raumtemperatur gab man 360 mg (1,1 mmol) Alkohol 5a in 3 ml DMF zu und anschließend 152 mg (1,2 mmol) Benzylchlorid in wenig DMF. Man ließ 18 h bei Raumtemp. rühren, versetzte mit Wasser und extrahierte dreimal mit Toluol. Trocknen der vereinigten Toluolphasen über Magnesiumsulfat, Abziehen des Lösungsmittel im Vakuum und Chromatographie auf Kieselgel (Essigester) ergab 200 mg (0,5 mmol, 45 %) Benzylether 6a als farblose Flüssigkeit (DC, EE/$CH_3OH$ = 10 : 1, Rf = 0,36)

$^1$H-NMR (60 MHz, $CDCl_3$) $\delta$ = 0,66 - 2,0 (m, 9H, - $(CH_2)_3CH_3$), 3,33 - 3,66 (m, 4H, -$H_2COCH_2$-), 3,93 - 4,41 (m, 1H, Methin-H), 4,58 (d, 2H, -$OCH_2$-Ph), 5,08 (s, 2H, Im-$CH_2$-Ph), 5,9 - 7,6 (m,14H, arom. H und olefin H)

In Analogie zu Beispiel 7a wurden weitere u. a. die in Tab. 4 aufgeführten Beispiel 7b - 7f erhalten.

**Tabelle 4**

| Beispiel | 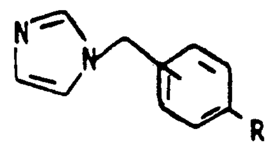 R = | charakteristische $^{1}$H-NMR-Signale, $\delta$= | Rf Kieselgel |
|---|---|---|---|
| 7b | 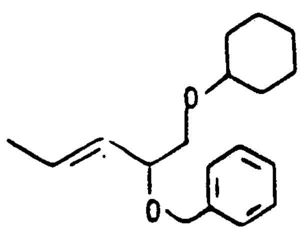 para | 1,0-2,1(m,10H, Cyclohexanmethylen-H), 3,4 (m,1H,Methin-H), 3,6(d,2H,$OCH_2$-), 4,1(m,1H,Methin-H),4,6(d,2H,Ph-$CH_2$O-),5,1(s,2H, N-$CH_2$-),6,0-7,6 (m,14H, arom. H und olefin H) | 0,26 $CHCl_3$ / $CH_3OH$ = 20:2 |
| 7c | 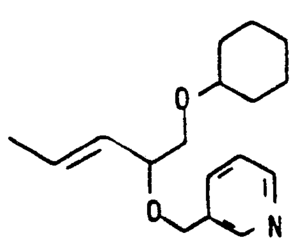 para | 1,0-2,1(m,10H, Cyclohexanmethylen-H),3,4 (m,1H,Methin-H), 3,6(d,2H,$OCH_2$-), 4,2(m,1H,Methin-H), 4,6 ("s", 2H,Ph-$CH_2$O),5,1 (s,2H,N-$CH_2$), 6,0-8,7(m, arom. H und olefin. H) | 0,14 Essigester / $CH_3OH$ = 20:2 |

| | | | |
|---|---|---|---|
| 7d | 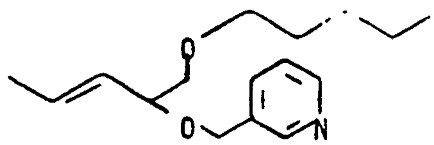 para | 0,7-1,9(m,9H, $-(CH_2)_3CH_3$),3,3-3,7 (m,4H, $-CH_2OCH_2-$),4,0-4,5 (m,1H,Methin-H), 4,6("s",2H, $-OCH_2$Pyr) | 0,17 Essigester/ $CH_3OH$ = 10:1 |
| 7e | 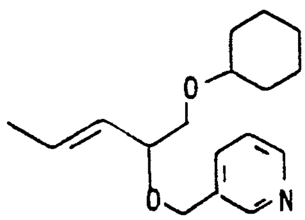 ortho | 1,0-2,0(m,10H, Cyclohexanmethylen-H), 3,4 (m,1H,Methin-H), 3,6(d,2H,$OCH_2-$), 4,1(m,1H,Methin-H), 4,6("s",2H Pyr$CH_2O-$) | 0,18 Essigester/ $CH_3OH$ = 20:1 |
| 7f | 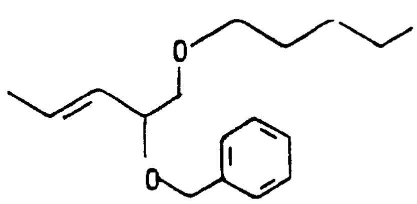 meta | 0,66-2,0(m,9H, $-(CH_2)_3CH_3$), 3,3-3,6 (m,4H, $-CH_2OCH_2-$), 3,9-4,4(m,1H, Methin-H), 4,6(d,2H, $-OCH_2$Ph) | 0,4 Essigester/ $CH_3OH$ = 10:1 |

**Patentansprüche** für die Vertragsstaate: BE, CH, DE, FR, GB, IT, LT, LU, NL, SE

1. Verbindungen der Formel I

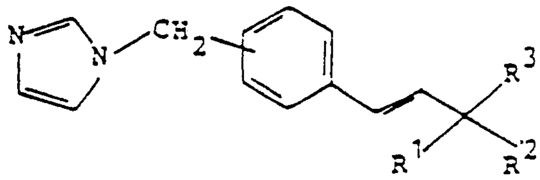

I

o, m, p

vorin bedeuten:

$R^1$ und $R^2$ zusammen mit dem sie tragenden Kohlenstoffatomen die Carbonylgruppe, oder $R^1$ Wasserstoff und $R^2$ den Rest $-OR^4$, worin $R^4$ Wasserstoff darstellt oder

a) einen verzweigten oder unverzweigten aliphatischen Acylrest mit bis zu 10 C-Atomen,

b) den Benzylcarbonyl- oder Benzoylrest, wobei der Phenylrest unsubstituiert oder 1- bis 3-fach substituiert ist mit Halogen oder $C_1$ - $C_4$-Alkyl,

c) den Rest 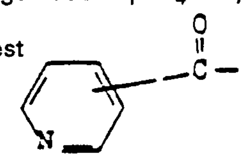

d) verzweigtes oder unverzweigtes Alkyl mit 1 - 10 C-Atomen,

e) den Benzylrest, wobei der Phenylkern unsubstituiert oder 1- bis 3-fach substituiert ist mit Halogen oder $C_1$ - $C_4$-Alkyl, oder

f) den Rest 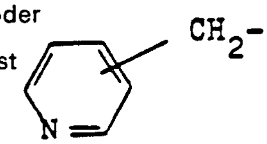

$R^3$ einen Phenylrest, der im Kern 1- bis 3-fach substituiert sein kann mit Halogen, Trifluormethyl und/oder Alkyl oder Alkoxy mit je 1 - 4 C-Atomen, oder einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3 bis 7 C-Atomen, einem unsubstituierten Phenyl-, α- oder β-Thienyl oder α- oder β-Furylrest, welche ihrerseits im Kern 1-bis 3-fach substituiert sind mit Halogen, Trifluormethyl und/oder Alkyl oder Alkoxy mit 1 bis 4 C-Atomen,

c) einem unsubstituierten Phenoxy-, α- oder β-Thienyloxy oder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen,

d) einem 1-Imidazolylrest.

2. Verbindungen der Formel I nach Anspruch 1, worin bedeuten

$R^1$ und $R^2$ zusammen mit dem sie tragenden Kohlenstoffatom die Carbonylgruppe, oder $R^1$ Wasserstoff und $R^2$ den Rest $-OR^4$, worin $R^4$ Wasserstoff darstellt oder

a) verzweigtes oder unverzweigtes Alkanoyl mit bis zu 6 C-Atomen,

b) den Benzylcarbonyl- oder Benzoylrest, wobei der Phenylkern unsubstituiert oder einfach substituiert ist mit Fluor, Chlor oder Methyl,

c) den Rest 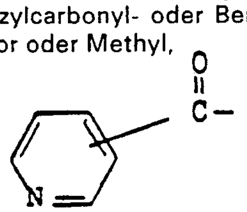

d) verzweigtes oder unverzweigtes Alkyl mit 1 - 6 C-Atomen,

e) den Benzylrest, wobei der Phenylkern unsubstituiert oder einfach substituiert ist mit Chlor, Fluor oder Methyl, oder

f) den Rest 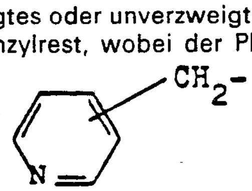

$R^3$ einen cycloaliphatischen Rest mit 3 - 8 Kohlenstoffatomen, einen geradkettigen oder verzweigten

Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Cycloalkyl mit 3 - 7 C-Atomen, einem unsubstituierten Phenyl-, α- oder β-Thienyl oder α- oder β-Furylrest,

c) einem unsubstituierten Phenoxy-, α- oder β-Thienyloxy oder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen, oder

d) einem 1-Imidazolylrest

3. Verbindungen der Formel I nach Anspruch 1, worin bedeuten:

$R^1$ und $R^2$ zusammen mit dem sie tragenden Kohlenstoffatom die Carbonylgruppe, oder $R^1$ Wasserstoff und $R^2$ denn Rest $-OR^4$, worin $R^4$ Wasserstoff, Benzyl, Alkyl mit 1 - 6 C-Atomen oder

Rest 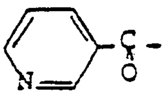

oder 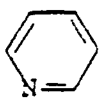 $-CH_2-$ darstellt,

$R^3$ einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits subtituiert sein können mit

a) einem geradkettigen oder verzweigten Alkoxyrest mit bis zu 10 Kohlenstoffatomen

b) Cycloalkyl mit 3 bis 7 C-Atomen, einem unsubstituierten Phenyl-, α- oder β-Thienyl oder α oder β-Furylrest

c) einem unsubstituierten Phenoxy-, α- oder β-Thienyloxy oder Cycloalkoxyrest mit 3 - 7 Kohlenstoffatomen oder

d) einem 1-Imidazolylrest.

4. Verfahren zur Herstellung von Verbindungen der Formel I gemäß Anspruch 1 dadurch gekennzeichnet, daß man,

a) die entsprechenden ortho-, meta- und parasubstituierten 1-Imidazolylmethylbenzaldehyde der Formel II

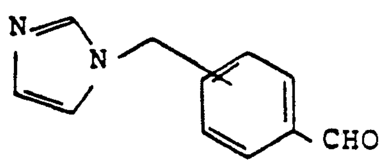

II

nach Horner-Emmons-Wittig mit einem Phosphonsäureester der allgemeinen Formel III,

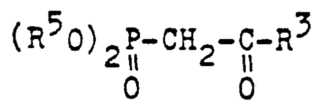

III

wobei $R^3$ die in der allgemeinen Formel I angebene Bedeutung besitzt und $R^5$ $C_1$-$C_4$-Alkyl bedeutet, zu Verbindungen der allgemeinen Formel IV umsetzt,

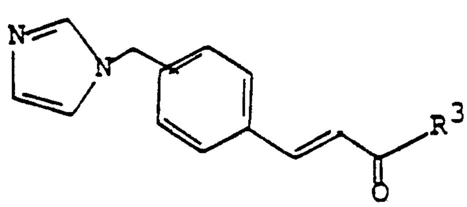

IV

wobei $R^3$ die zur Formel I angegebene Bedeutung hat,

b) gegebenenfalls die Enone der allgemeinen Formel IV mit einem Reduktionsmittel zu den Alkoholen der allgemeinen Formel V reduziert,

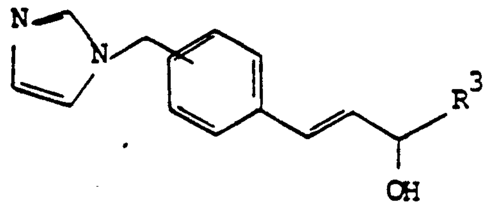

V

worin $R^3$ die zur Formel I gegebene Bedeutung hat,

c) gegebenenfalls die Alkohole der Formel V in Form ihrer Racemate oder als reine Enantiomere mit einem reaktionsfähigen Derivat einer Carbonsäure zu den Estern der allgemeinen VI umsetzt,

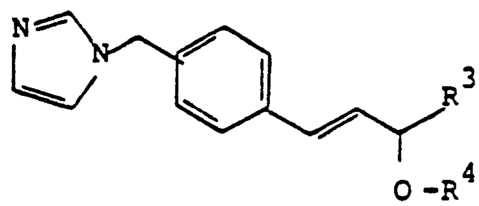

VI

worin $R^3$ die zur Formel I gegebene Bedeutung hat, und $R^4$ einen verzweigten oder unverzweigten aliphatischen Acylrest mit bis zu 10 C-Atomen, oder den Benzylcarbonyl- oder Benzoylrest, worin der Phenylkern 1- bis 3-fach mit Halogen oder $C_1$-$C_4$-Alkyl substituiert sein kann, oder den Rest darstellt,

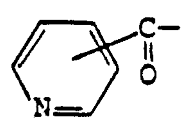

d) gegebenenfalls die Alkohole der Formel V in Form ihrer Racemate oder als reine Enantiomere mit einem entsprechenden Halogenid, Tosylat oder Mesylat zu den Ethern der allgemeinen Formel VII umsetzt,

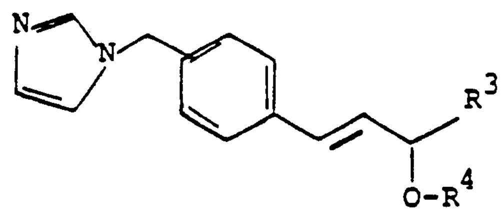

VII

worin $R^4$ $C_1$-$C_{10}$-Alkyl, Benzyl, worin der Phenylkern 1- bis 3-fach mit Halogen oder $C_1$-$C_4$-Alkyl substituiert sein kann, oder den Rest

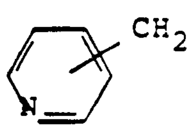

darstellt.

5. Arzneimittel, gekennzeichnet durch den Gehalt an einer Verbindung der Formel I gemäß Anspruch 1 in Mischung mit einem pharmazeutisch üblichen Träger und/ oder Stabilisator.

6. Verwendung einer Verbindung der Formel I oder eines pharmazeutisch unbedenklichen Säureadditionssalzes zur Herstellung von Arzneimitteln zur Behandlung von Krankheiten mit erhöhter Thrombozytenaggregationsneigung.

**Patentansprüche** fü den Vertragsstaat: AT

1. Verfahren zur Herstellung von Verbindungen der Formel I,

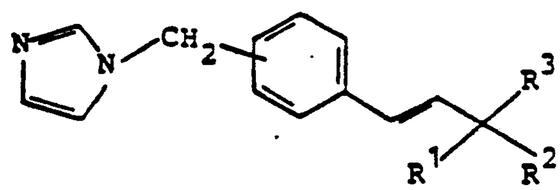

I

o, m, p

worin bedeuten:

$R^1$ und $R^2$ zusammen mit dem sie tragenden Kohlenstoffatom die Carbonylgruppe, oder $R^1$ Wasserstoff und $R^2$ den Rest $-OR^4$, worin $R^4$ Wasserstoff darstellt oder

a) einen verzweigten oder unverzweigten aliphatischen Acylrest mit bis zu 10 C-Atomen,

b) den Benzylcarbonyl- oder Benzoylrest, wobei der Phenylrest unsubstituiert oder 1- bis 3-fach substituiert ist mit Halogen oder $C_1$-$C_4$-Alkyl,

c) den Rest

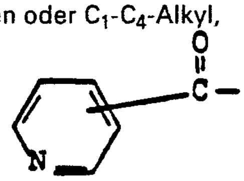

d) verzweigtes oder unverzweigtes Alkyl mit 1 - 10 C-Atomen,

e) den Benzylrest, wobei der Phenylkern unsubstituiert oder 1- bis 3-fach substituiert ist mit Halogen oder $C_1$-$C_4$-Alkyl, oder

f) den Rest

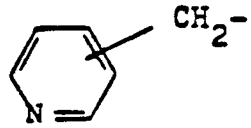

$R^3$ einen Phenylrest, der im Kern 1- bis 3-fach substituiert sein kann mit Halogen, Trifluormethyl und/oder Alkyl oder Alkoxy mit je 1 - 4 C-Atomen, oder einen cycloaliphatischen Rest mit 3 - 8 Kohlenstoffatomen, einen geradkettigen oder verzweigten Alkylrest-mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3 bis 7 C-Atomen, einem unsubstituierten Phenyl-, α- oder β-Thienyl oder α- oder β-Furylrest, welche ihrerseits im Kern 1-bis 3-fach substituiert sind mit Halogen, Trifluormethyl und/oder Alkyl oder Alkoxy mit 1 bis 4 C-Atomen,

c) einem unsubstituierten Phenoxy-, α- oder β-Thienyloxy oder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen,

d) einem 1-Imidazolylrest dadurch gekennzeichnet, daß man

a) die entsprechenden ortho-, meta- und parasubstituierten 1-Imidazolylmethylbenzaldehyde der Formel II

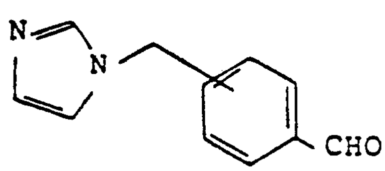

II

nach Horner-Emmons-Wittig mit einem Phosphonsäureester der allgemeinen Formel III,

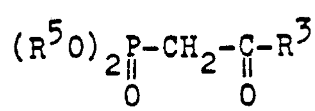

III

wobei $R^3$ die in der allgemeinen Formel I angegebene Bedeutung besitzt und $R^5$ $C_1$-$C_4$-Alkyl bedeutet, zu Verbindungen der allgemeinen Formel IV umsetzt,

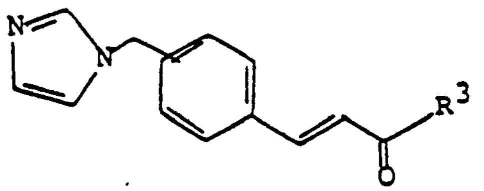

IV

wobei $R^3$ die zur Formel I angegebene Bedeutung hat,

b) gegebenenfalls die Enone der allgemeinen Formel IV mit einem Reduktionsmittel zu den Alkoholen der allgemeinen Formel V reduziert,

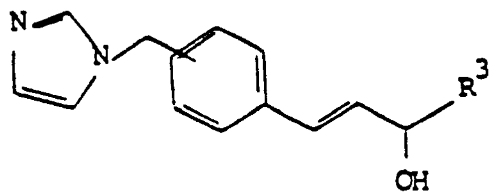

V

worin $R^3$ die zur Formel I gegebene Bedeutung hat,

c) gegebenenfalls die Alkohole der Formel V in Form ihrer Racemate oder als reine Enantiomere mit einem reaktionsfähigen Derivat einer Carbonsäure zu den Estern der allgemeinen Formel VI umsetzt,

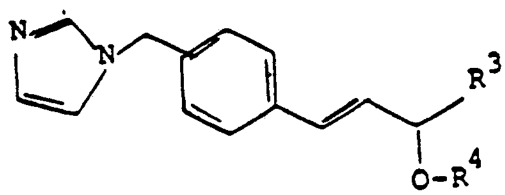

VI

worin $R^3$ die zur Formel I gegebene Bedeutung hat und $R^4$ einen verzweigten oder unverzweigten aliphatischen Acylrest mit bis zu 10 C-Atomen, oder den Benzylcarbonyl- oder Benzoylrest, worin der Phenylkern 1- bis 3-fach mit Halogen oder $C_1$-$C_4$-Alkyl substituiert sein kann, oder den Rest 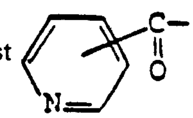 darstellt,

d) gegebenenfalls die Alkohole der Formel V in Form ihrer Racemate oder als reine Enantiomere mit einem entsprechenden Halogenid, Tosylat oder Mesylat zu den Ethern der allgemeinen Formel VII umsetzt,

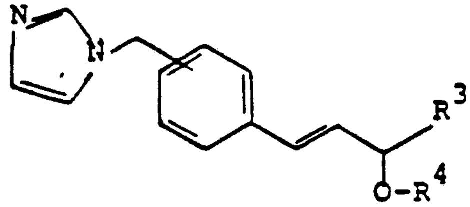

VII

worin $R^4$ $C_1$-$C_{10}$-Alkyl, Benzyl, worin der Phenylkern 1- bis 3-fach mit Halogen oder $C_1$-$C_4$-Alkyl substituiert sein kann, oder den Rest

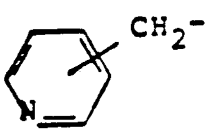 darstellt.

2. Verfahren gemäß Anspruch 1, dadurch gekennzeichnet, daß die Substituenten in Formel I bedeuten.

$R^1$ und $R^2$ zusammen mit dem sie tragenden Kohlenstoffatom die Carbonylgruppe, oder $R^1$ Wasserstoff und $R^2$ den Rest -$OR^4$, worin $R^4$ Wasserstoff darstellt oder

a) verzweigtes oder unverzweigtes Alkanoyl mit bis zu 6 C-Atomen,

b) den Benzylcarbonyl- oder Benzoylrest, wobei der Phenylkern unsubstituiert oder einfach substituiert ist mit Fluor, Chlor oder Methyl,

c) den Rest

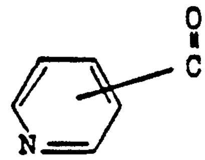

d) verzweigtes oder unverzweigtes Alkyl mit 1 - 6 C-Atomen,

e) den Benzylrest, wobei der Phenylkern unsubstituiert oder einfach substituiert ist mit Chlor, Fluor oder Methyl, oder

f) den Rest

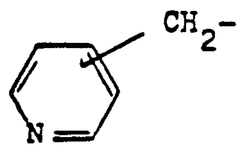

$R^3$ einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Cycloalkyl mit 3 - 7 C-Atomen, einem unsubstituierten Phenyl-, α- oder β-Thienyl oder α- oder β-Furylrest,

c) einem unsubstituierten Phenoxy-, α- oder β-Thienyloxy oder Cycloalkoxyrest mit 3 - 7 Kohlenstoffatomen oder einem der genannten Reste, welcher seinerseits im Kern 1- bis 3-fach substituiert ist mit Halogen, Trifluormethyl und/oder Alkyl oder Alkoxy mit je 1 - 4 C-Atomen, oder

d) einem 1-Imidazolylrest.

3. Verfahren gemäß Anspruch 1, dadurch gekennzeichnet, daß die substituenten in Formel I bedeuten:

$R^1$ und $R^2$ zusammen mit dem sie tragenden Kohlenstoffatom die Carbonylgruppe, oder $R^1$ Wasserstoff und $R^2$ den Rest $-OR^4$, worin $R^4$ Wasserstoff, Benzyl, Alkyl mit 1 - 6 C-Atomen oder den Rest

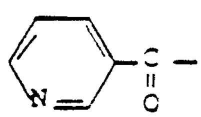

oder 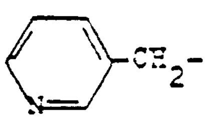 darstellt,

$R^3$ einen cycloaliphatischen Rest mit 3 - 8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen

b) Cycloalkyl mit 3 bis 7 C-Atomen, einem unsubstituierten Phenyl-, α- oder β-Thienyl oder α- oder β-Furylrest oder einem Phenyl-, Thienyl- oder Furylrest,

c) einem unsubstituierten Phenoxy-, α- oder β-Thienyloxy oder Cycloalkoxyrest mit 3 - 7 Kohlenstoffatomen oder

d) einem 1-Imidazolylrest.

**Claims** for the contracting states: BE, CH, DE, FR, GB, IT, LI, LU, NL, SE

1. A compound of the formula I

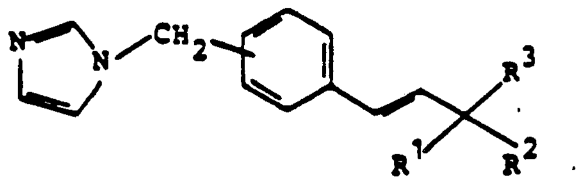

I

o, m, p

in which:

$R^1$ and $R^2$, together with the carbon atom which carries them, denote the carbonyl group, or

$R^1$ denotes hydrogen, and $R^2$ denotes the radical $-OR^4$ in which $R^4$ represents hydrogen, or

a) a branched or unbranched aliphatic acyl radical having up to 10 carbon atoms,

b) the benzylcarbonyl or benzoyl radical, with the phenyl radical being unsubstituted or substituted once to three times with halogen or $C_1$-$C_4$-alkyl,

c) the radical 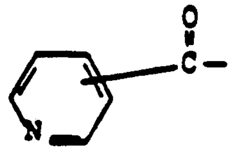

d) branched or unbranched alkyl having 1 - 10 carbon atoms,

e) the benzyl radical, with the phenyl nucleus being unsubstituted or substituted once to three times with halogen or $C_1$-$C_4$-alkyl, or

f) the radical 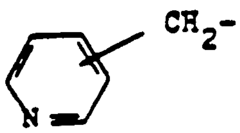

$R^3$ denotes a phenyl radical which can be substituted once to three times in the nucleus with halogen, trifluoromethyl and/or alkyl or alkoxy, each having 1 - 4 carbon atoms, or a cycloaliphatic radical having 3 - 8 carbon atoms, a straight-chain or branched alkyl radical having up to 8 carbon atoms, a straight-chain or branched unsaturated aliphatic hydrocarbon radical having 3 to 8 carbon atoms, it being possible for the aliphatic radicals in turn to be substituted with

a) a straight-chain or branched alkoxy radical having up to 10 carbon atoms, or a cycloalkoxy radical having up to 6 carbon atoms, or a straight-chain or branched alkenyloxy or alkynyloxy radical having 3 to 6 carbon atoms,

b) halogen, cycloalkyl having 3 to 7 carbon atoms, an unsubstituted phenyl, α- or β-thienyl or α- or β-furyl radical, or a phenyl, thienyl, or furyl radical which in turn are substituted once to three times in the nucleus with halogen, trifluoromethyl and/or alkyl or alkoxy having 1 - 4 carbon atoms,

c) an unsubstituted phenoxy, α- or β-thienyloxy or cycloalkoxy radical having 3 - 7 carbon atoms, or one of the radicals mentioned which in turn is substituted once to three times in the nucleus with halogen, trifluoromethyl and/or alkyl or alkoxy each having 1 - 4 carbon atoms, or

d) a 1-imidazolyl radical.

2. A compound of the formula I as claimed in claim 1,

in which

$R^1$ and $R^2$, together with the carbon atom which carries them, denote the carbonyl group, or $R^1$ denotes hydrogen and $R^2$ denotes the radical $-OR^4$ in which $R^4$ represents hydrogen, or

a) branched or unbranched alkanoyl having up to 6 carbon atoms,

b) the benzylcarbonyl or benzoyl radical, with the phenyl nucleus being unsubstituted or substituted once with fluorine, chlorine or methyl,

c) the radical

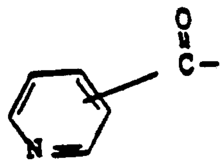

d) branched or unbranched alkyl having 1 - 6 carbon atoms,
e) the benzyl radical, with the phenyl nucleus being unsubstituted or substituted once with chlorine, fluorine or methyl, or
f) the radical

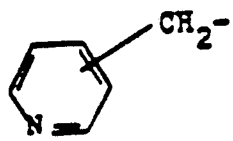

,

$R^3$ denotes a cycloaliphatic radical having 3 - 8 carbon atoms, a straight-chain or branched alkyl radical having up to 8 carbon atoms, a straight-chain or branched unsaturated aliphatic hydrocarbon radical having 3 to 8 carbon atoms, it being possible for the aliphatic radicals in turn to be substituted with
a) a straight-chain or branched alkoxy radical having up to 6 carbon atoms, or a cycloalkoxy radical having up to 6 carbon atoms, or a straight-chain or branched alkenyloxy or alkyoxy radical having 3 to 6 carbon atoms,
b) cycloalkyl having 3 - 7 carbon atoms, an unsubstituted phenyl, α- or β-thienyl or α- or β-furyl radical,
c) an unsubstituted phenoxy, α- or β-thienyloxy or cycloalkoxy radical having 3 - 7 carbon atoms, or one of the radicals mentioned which in turn is substituted once to three times in the nucleus with halogen, trifluoromethyl and/or alkyl or alkoxy each having 1 - 4 carbon atoms, or
d) a 1-imidazolyl radical.

3. A compound of the formula I as claimed in claim 1,
in which
$R^1$ and $R^2$, together with the carbon atom which carries them, denote the carbonyl group, or $R^1$ denotes hydrogen, and $R^2$ denotes the radical $-OR^4$ in which $R^4$ represents hydrogen, benzyl, alkyl having 1 - 6 carbon atoms or the radical

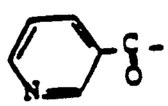

or

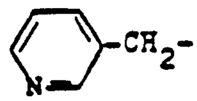

,

$R^3$ denotes a cycloaliphatic radical having 3 - 8 carbon atoms, a straight-chain or branched alkyl radical having up to 8 carbon atoms, a straight-chain or branched unsaturated aliphatic hydrocarbon radical having 3 to 8 carbon atoms, it being possible for the aliphatic radicals in turn to be substituted with
a) a straight-chain or branched alkoxy radical having up to 10 carbon atoms,
b) cycloalkyl having 3 to 7 carbon atoms, an unsubstituted phenyl, α- or β-thienyl or α- or β-furyl radical,
c) an unsubstituted phenoxy, α- or β-thienyloxy or cycloalkoxy radical having 3 - 7 carbon atoms, or
d) a 1-imidazolyl radical.

4. A process for the preparation of compounds of the formula I as claimed in claim 1, characterized by
a) reacting the appropriate ortho-, meta- and parasubstituted 1-imidazolylmethylbenzaldehydes of the formula II

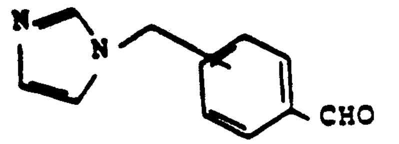

II

by the method of Horner-Emmons-Wittig with a phosphonic ester of the general formula III

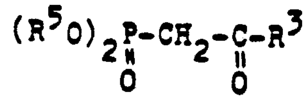

$R^3$ having the meaning indicated in the general formula I, and $R^5$ denoting $C_1$-$C_4$-alkyl, to give the compounds of the general formula IV

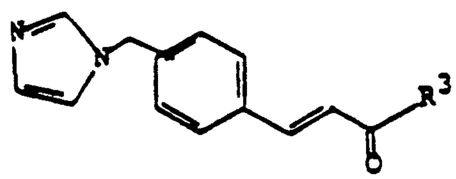

IV

$R^3$ having the meaning indicated for formula I b) where appropriate reducing the enones of the general formula IV with a reducing agent to give the alcohols of the general formula V

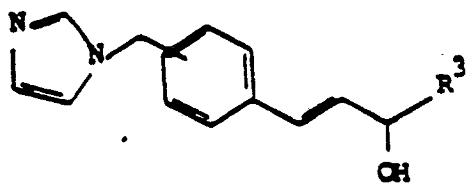

V

in which $R^3$ has the meaning given for formula I,

c) where appropriate reacting the alcohols of the formula V, in the form of their racemates or as pure enantiomers, with a reactive derivative of a carboxylic acid to give the esters of the general formula VI

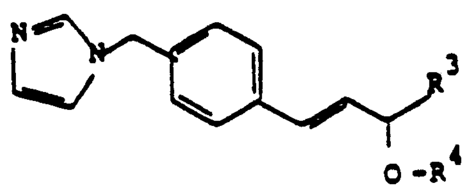

VI

in which $R^3$ has the meaning given for formula I, and $R^4$ represents a branched or unbranched aliphatic acyl radical having up to 10 carbon atoms, or the benzylcarbonyl or benzoyl radical, in which the phenyl nucleus can be substituted once to three times with halogen or $C_1$-$C_4$-alkyl, or the radical

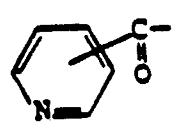 ,

or

d) where appropriate reacting the alcohols of the formula V, in the form of their racemates or as pure enantiomers, with an appropriate halide, tosylate or mesylate, to give the ethers of the general formula VII

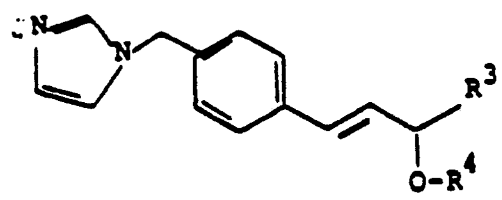

VII

in which $R^4$ represents $C_1$-$C_{10}$-alkyl, benzyl in which the phenyl nucleus can be substituted once to three times with halogen or $C_1$-$C_4$-alkyl, or the radical

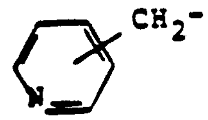

5. A medicament characterized by containing a compound of the formula I, as claimed in claim 1, in mixture with a permaceutically customary vehicle and/or stabilizer.

6. The use of a compound of the formula I, or of a pharmaceutically acceptable acid addition salt, for the preparation of medicaments for the treatment of disorders where the tendency to platelet aggregation is increased.

**Claims** for the contracting state: AT

1. A process for the preparation of a compound of the formula I

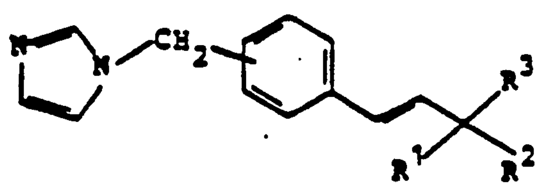

I

o, m, p

in which:

$R^1$ and $R^2$, together with the carbon atom which carries them, denote the carbonyl group, or

$R^1$ denotes hydrogen, and $R^2$ denotes the radical $-OR^4$ in which $R^4$ represents hydrogen, or

a) a branched or unbranched aliphatic acyl radical having up to 10 carbon atoms,

b) the benzylcarbonyl or benzoyl radical, with the phenyl radical being unsubstituted or substituted once to three times with halogen or $C_1$-$C_4$-alkyl,

c) the radical

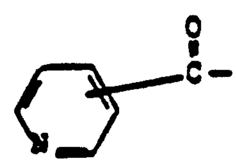

d) branched or unbranched alkyl having 1 - 10 carbon atoms,

e) the benzyl radical, with the phenyl nucleus being unsubstituted or substituted once to three times with halogen or $C_1$-$C_4$-alkyl, or

f) the radical

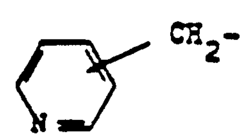

$R^3$ denotes a phenyl radical which can be substituted once to three times in the nucleus with halogen, trifluoromethyl and/or alkyl or alkoxy, each having 1-4 carbon atoms, or a cycloaliphatic radical having 3 - 8 carbon atoms, a straight-chain or branched alkyl radical having up to 8 carbon atoms, a straight-chain or

branched unsaturated aliphatic hydrocarbon radical having 3 to 8 carbon atoms, it being possible for the aliphatic radicals in turn to be substituted with

a) a straight-chain or branched alkoxy radical having up to 10 carbon atoms, or a cycloalkoxy radical having up to 6 carbon atoms, or a straight-chain or branched alkenyloxy or alkynyloxy radical having 3 to 6 carbon atoms,

b) halogen, cycloalkyl having 3 to 7 carbon atoms, an unsubstituted phenyl, α- or β-thienyl or α- or β-furyl radical, or a phenyl, thienyl, or furyl radical which in turn are substituted once to three times in the nucleus with halogen, trifluoromethyl and/or alkyl or alkoxy having 1 - 4 carbon atoms,

c) an unsubstituted phenoxy, α- or β-thienyloxy or cycloalkoxy radical having 3 - 7 carbon atoms, or one of the radicals mentioned which in turn is substituted once to three times in the nucleus with halogen, trifluoromethyl and/or alkyl or alkoxy each having 1 - 4 carbon atoms, or

d) a 1-imidazolyl radical characterized by

a) reacting the appropriate ortho-, meta- and parasubstituted 1-imidazolylmethylbenzaldehydes of the formula II

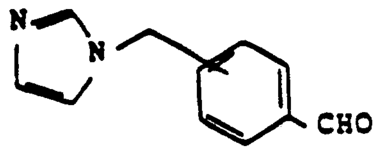

II

by the method of Horner-Emmons-Wittig with a phosphonic ester of the general formula III

$$(R^5O)_2\overset{\displaystyle O}{\overset{\|}{P}}-CH_2-\overset{\displaystyle O}{\overset{\|}{C}}-R^3 \qquad III$$

$R^3$ having the meaning indicated in the general formula I, and $R^5$ denoting $C_1$-$C_4$-alkyl, to give the compounds of the general formula IV

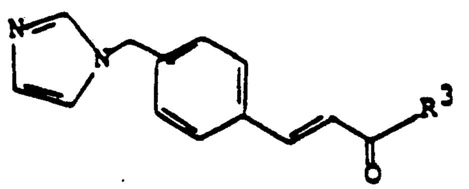

IV

$R^3$ having the meaning indicated for formula I

b) where appropriate reducing the enones of the general formula IV with a reducing agent to give the alcohols of the general formula V

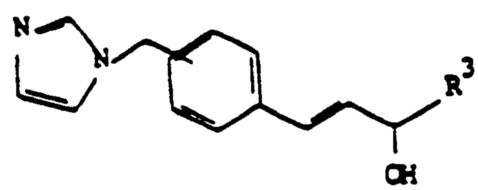

V

in which $R^3$ has the meaning given for formula I,

c) where appropriate reacting the alcohols of the formula V, in the form of their racemates or as pure enantiomers, with a reactive derivative of a carboxylic acid to give the esters of the general formula VI

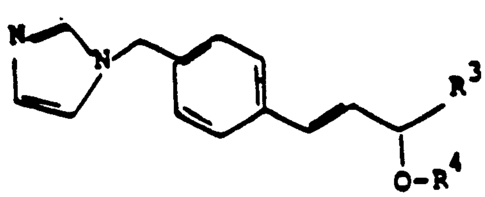

VI

in which $R^3$ has the meaning given for formula I, and $R^4$ represents a branched or unbranched aliphatic acyl radical having up to 10 carbon atoms, or the benzylcarbonyl or benzoyl radical, in which the phenyl nucleus can

be substituted once to three times with halogen or $C_1$-$C_4$-alkyl, or the radical

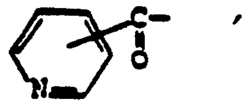

d) where appropriate reacting the alcohols of the formula V, in the form of their racemates or as pure enantiomers, with an appropriate halide, tosylate or mesylate, to give the ethers of the general formula VII

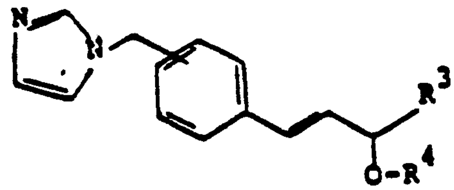

VII

in which $R^4$ represents $C_1$-$C_{10}$-alkyl, benzyl in which the phenyl nucleus can be substituted once to three times with halogen or $C_1$-$C_4$-alkyl, or the radical

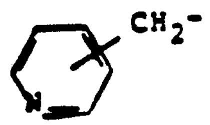

2. The process as claimed in claim 1, characterized in that in formula I the substituents

$R^1$ and $R^2$, together with the carbon atom which carries them, denote the carbonyl group, or $R^1$ denotes hydrogen and $R^2$ denotes the radical $-OR^4$ in which $R^4$ represents hydrogen, or

a) branched or unbranched alkanoyl having up to 6 carbon atoms,

b) the benzylcarbonyl or benzoyl radical, with the phenyl nucleus being unsubstituted or substituted once with fluorine, chlorine or methyl,

c) the radical

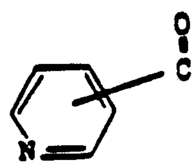

d) branched or unbranched alkyl having 1 - 6 carbon atoms,

e) the benzyl radical, with the phenyl nucleus being unsubstituted or substituted once with chlorine, fluorine or methyl, or

f) the radical

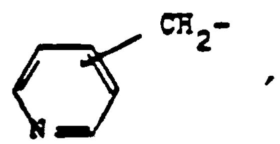

$R^3$ denotes a cycloaliphatic radical having 3 - 8 carbon atoms, a straight-chain or branched alkyl radical having up to 8 carbon atoms, a straight-chain or branched unsaturated aliphatic hydrocarbon radical having 3 to 8 carbon atoms, it being possible for the aliphatic radicals in turn to be substituted with

a) a straight-chain or branched alkoxy radical having up to 6 carbon atoms, or a cycloalkoxy radical having up to 6 carbon atoms, or a straight-chain or branched alkenyloxy or alkynyloxy radical having 3 to 6 carbon atoms,

b) cycloalkyl having 3 - 7 carbon atoms, an unsubstituted phenyl, α- or β-thienyl or α- or β-furyl radical,

c) an unsubstituted phenoxy, α- or β-thienyloxy or cycloalkoxy radical having 3 - 7 carbon atoms, or one of the radicals mentioned which in turn is substituted once to three times in the nucleus with halogen, trifluoromethyl and/or alkyl or alkoxy each having 1 - 4 carbon atoms, or

d) a 1-imidazolyl radical.

3. The process as claimed in claim 1, characterized in that in formula I the substituents

$R^1$ and $R^2$, together with the carbon atom which carries them, denote the carbonyl group, or $R^1$ denotes hydrogen, and $R^2$ denotes the radical $-OR^4$ in which $R^4$ represents hydrogen, benzyl, alkyl having 1 - 6 carbon

atoms or the radical

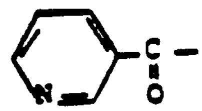

or

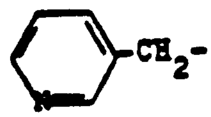 ,

$R^3$ denotes a cycloaliphatic radical having 3 - 8 carbon atoms, a straight-chain or branched alkyl radical having up to 8 carbon atoms, a straight-chain or branched unsaturated aliphatic hydrocarbon radical having 3 to 8 carbon atoms, it being possible for the aliphatic radicals in turn to be substituted with

a) a straight-chain or branched alkoxy radical having up to 10 carbon atoms,

b) cycloalkyl having 3 to 7 carbon atoms, an unsubstituted phenyl, α- or β-thienyl or α- or β-furyl radical,

c) an unsubstituted phenoxy, α- or β-thienyloxy or cycloalkoxy radical having 3 - 7 carbon atoms, or

d) a 1-imidazolyl radical.

**Revendications** pour les états contractants: BE, CH, DE, FR, GB, IT, LI, LU, NL, SE

1. Composés de formule I

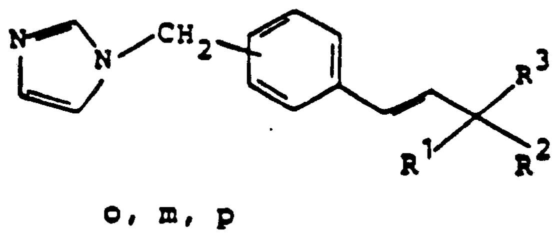

I

dans laquelle:

$R^1$ et $R^2$ représentent, avec l'atome de carbone qui les porte, le groupe carbonyle, ou $R^1$ représente l'hydrogene et $R^2$ le radical $-OR^4$, $R^4$ représentant l'hydrogène ou

a) un radical acyle aliphatique ramifié ou non ramifié ayant jusqu'à 10 atomes de carbone,

b) le radical benzylcarbonyle ou benzoyle dont le radical phényle est non substitué ou substitué 1 à 3 fois par un halogène ou un radical alkyle en $C_1$-$C_4$,

c) le radical

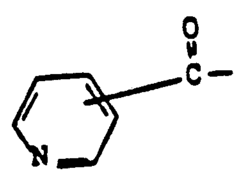

d) un radical alkyle ramifié ou non ramifié ayant 1 à 10 atomes de carbone,

e) le radical benzyle, dont le noyau phényle est non substitué ou substitué 1 à 3 fois par un halogène ou un radical alkyle en $C_1$-$C_4$, ou

f) le radical

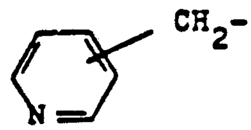

$R^3$ représente un radical phényle, qui peut être substitué 1 à 3 fois sur le noyau par un halogène, un radical trifluorométhyle et/ou alkyle ou alkoxy ayant chacun 1 à 4 atomes de carbone, ou un radical cycloaliphatique ayant 3 à 8 atomes de carbone, un radical alkyle linéaire ou ramifié ayant jusqu'à 8 atomes de carbone, un radical hydrocarboné aliphatique insaturé linéaire ou ramifié ayant 3 à 8 atomes de carbone, les radicaux aliphatiques pouvant de leur côté être substitués par:

a) un radical alkoxy linéaire ou ramifié ayant jusqu'à 10 atomes de carbone ou un radical cycloalkoxy ayant jusqu'à 6 atomes de carbone ou un radical alkényloxy ou alkynyloxy linéaire ou ramifié ayant 3 à 6 atomes de carbone,

b) un halogène, un radical cycloalkyle ayant 3 à 7 atomes de carbone, un radical phényle, α - ou β -thiényle ou α - ou β -furyle non substitué, qui, de leur côté, sont substitués sur le noyau 1 à 3 fois par un halogène, un radical trifluorométhyle et/ou un radical alkyle ou alkoxy ayant 1 à 4 atomes de carbone,

c) un radical phénoxy, α - ou β -thiényloxy ou cycloalkoxy ayant 3 à 7 atomes de carbone non substitué ou un des radicaux cités, qui, de son côté, est substitué sur le noyau 1 à 3 fois par un halogène, un radical trifluorométhyle et/ou un radical alkyle ou alkoxy ayant chacun 1 à 4 atomes de carbone,

d) un radical 1-imidazolyle.

2. Composés de formule I selon la revendication 1, dans laquelle:

$R^1$ et $R^2$ représentent, avec l'atome de carbone qui les porte, le groupe carbonyle, ou $R^1$ représente l'hydrogène et $R^2$ le radical $-OR^4$, $R^4$ representant l'hydrogène ou:

a) un radical alkanoyle ramifié ou non ramifié ayant jusqu'à 6 atomes de carbone,

b) le radical benzylcarbonyle ou benzoyle, dont le noyau phényle est non substitué ou substitué 1 fois par le fluor, le chlore ou un radical méthyle,

c) le radical

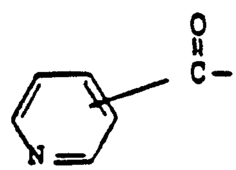

d) un radical alkyle ramifié ou non ramifié ayant 1 à 6 atomes de carbone,

e) le radical benzyle, dont le noyau phényle est non substitué ou substitué 1 fois par le chlore, le fluor ou un radical méthyle, ou

f) le radical

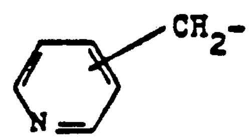

$R^3$ représente un radical cycloaliphatique ayant 3 à 8 atomes de carbone, un radical alkyle linéaire ou ramifié ayant jusqu'à 8 atomes de carbone, un radical hydrocarboné aliphatique insaturé linéaire ou ramifié ayant 3 à 8 atomes de carbone, les radicaux aliphatiques pouvant de leur côté être substitués par:

a) un radical alkoxy linéaire ou ramifié ayant jusqu'à 8 atomes de carbone ou un radical cycloalkoxy ayant jusqu'à 6 atomes de carbone ou un radical alkényloxy ou alkynyloxy linéaire ou ramifié ayant 3 à 6 atomes de carbone,

b) un radical cycloalkyle ayant 3 à 7 atomes de carbone, un radical phényle, α - ou β -thiényle ou α- ou β -furyle non substitué,

c) un radical phénoxy, α - ou β -thiényloxy ou cycloalkoxy ayant 3 à 7 atomes de carbone ou un des radicaux cités, qui de son côté est substitué sur le noyau 1 à 3 fois par un halogène, un radical trifluorométhyle et/ou un radical alkyle ou alkoxy ayant chacun 1 à 4 atomes de carbone ou

d) un radical 1-imidazolyle.

3. Composés de formule I selon la revendication 1, dans laquelle:

$R^1$ et $R^2$ représentent, avec l'atome de carbone qui les porte, le groupe carbonyle, ou $R^1$ représente l'hydrogène et $R^2$ le radical $-OR^4$, $R^4$ représentant l'hydrogène, un radical benzyle, un radical alkyle ayant 1 à 6 atomes de carbone ou le radical

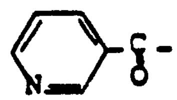

ou

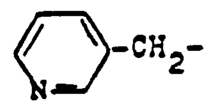

$R^3$ représente un radical cycloaliphatique ayant 3 à 8 atomes de carbone, un radical alkyle linéaire ou ramifié ayant jusqu'à 8 atomes de carbone, un radical hydrocarboné aliphatique insaturé linéaire ou ramifié ayant 3 à 8 atomes de carbone, les radicaux aliphatiques pouvant de leur côté être substitués par:

a) un radical alkoxy linéaire ou ramifié ayant jusqu'à 10 atomes de carbone,

b) un radical cycloalkyle ayant 3 à 7 atomes de carbone, un radical phényle, α - ou β -thiényle ou α - ou β -furyle non substitué

c) un radical phénoxy, α - ou β -thiényloxy ou cycloalkoxy ayant 3 à 7 atomes de carbone non substitué ou

d) un radical 1-imidazolyle.

4. Procédé de préparation de composés de formule I selon la revendication 1, caractérisé en ce que:

a) on fait réagir les 1-imidazolylméthylbenzaldéhydes ortho-, méta- et parasubstitués correspondants de formule II

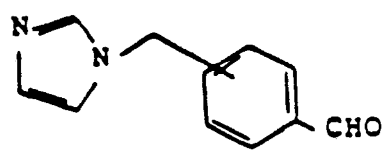

II

par la méthode de Horner-Emmons-Wittig avec un ester phosphonique de formule générale III,

$$\begin{array}{l}(R^5O)_2P-CH_2-C-R^3\\ \qquad\;\;\| \qquad\quad \|\\ \qquad\;\;O \qquad\quad O\end{array}$$

III

$R^3$ ayant la signification indiquée dans la formule générale I et $R^5$ representant un radical alkyle en $C_1$-$C_4$, pour obtenir des composés de formule générale IV,

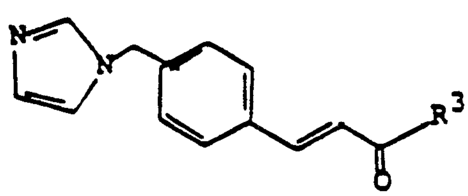

IV

$R^3$ ayant la signification indiquée pour la formule I,

b) éventuellement on réduit les énones de formule générale IV par un réducteur pour obtenir les alcools de formule générale V,

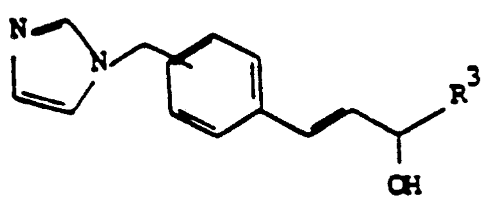

V

$R^3$ ayant la signification indiquée par la formule I,

c) éventuellement on fait réagir les alcools de formule V, sous la forme de leurs racémates ou sous la forme d'énantiomères purs, avec un dérivé réactif d'un acide carboxylique pour obtenir les esters de formule générale VI

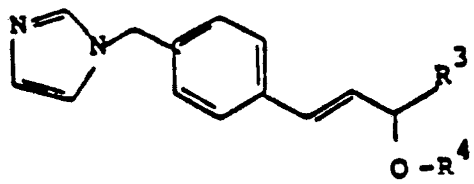

VI

dans laquelle $R^3$ a la signification indiquée pour la formule I et $R^4$ représente un radical acyle aliphatique ramifié ou non ramifié ayant jusqu'à 10 atomes de carbone ou le radical benzyl carbonyle ou benzoyle, le noyau phényle pouvant être substitué 1 à 3 fois par un halogène ou un radical alkyle en $C_1$-$C_4$, ou le radical

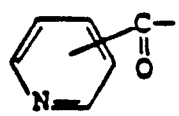

d) éventuellement on fait réagir les alcools de formule V, sous la forme de leurs racémates ou sous la forme d'énantiomères purs, avec un halogénure, un tosylate ou un mésylate correspondant pour obtenir les éthers de formule générale VII,

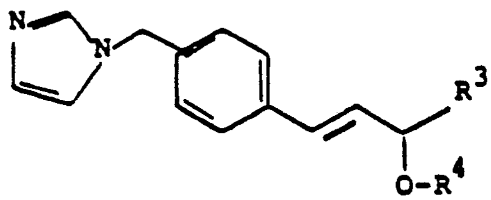

VII

dans laquelle $R^4$ représente un radical alkyle en $C_1$-$C_{10}$, un radical benzyle, dont le noyau phényle peut être substitué 1 à 3 fois par un halogène ou un radical alkyle en $C_1$-$C_4$, ou le radical

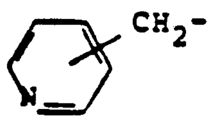

5. Médicament caractérisé en ce qu'il contient un composé de formule I selon la revendication 1 en mélange avec un support pharmaceutique classique et/ou à un stabilisant.

8. Utilisation d'un composé de formule I ou d'un de ses sels d'addition avec des acides pharmaceutiquement acceptables pour préparer des médicaments destinés à traiter les maladies dans lequelles il y a une tendance accrue à l'agrégation des thrombocytes.

**Revendications** pour l'éteat contractant: AT

1. Procédé de préparation des composés de formule I

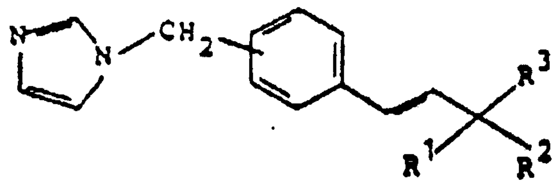

I

o, m, p

dans laquelle:

$R^1$ et $R^2$ représentent, avec l'atome de carbone qui les porte, le groupe carbonyle, ou $R^1$ représente l'hydrogène et R le radical $-OR^4$, $R^4$ représentant l'hydrogène ou

a) un radical acyle aliphatique ramifié ou non ramifié ayant jusqu'à 10 atomes de carbone,

b) le radical benzylcarbonyle ou benzoyle dont le radical phényle est non substitué ou substitué 1 à 3 fois par un halogène ou un radical alkyle en $C_1$-$C_4$,

c) le radical

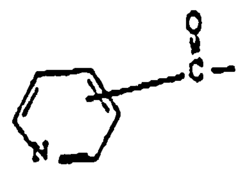

d) un radical alkyle ramifié ou non ramifié ayant 1 à 10 atomes de carbone,

e) le radical benzyle, dont le noyau phényle est non substitué ou substitué 1 à 3 fois par un halogène ou un radical alkyle en $C_1$-$C_4$, ou

f) le radical

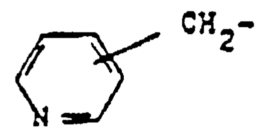

$R^3$ représente un radical phényle, qui peut être substitué 1 à 3 fois sur le noyau par un halogène, un radical trifluorométhyl, et/ou alkyle ou alkoxy ayant chacun 1 à 4 atomes de carbone, ou un radical cycloaliphatique ayant 3 à 8 atomes de carbone, un radical alkyle linéaire ou ramifié ayant jusqu'à 8 atomes de carbone, un radical hydrocarboné aliphatique insaturé linéaire ou ramifié ayant 3 à 8 atomes de carbone, les radicaux aliphatiques pouvant de leur côté être substitués par:

a) un radical alkoxy linéaire ou ramifié ayant jusqu'à 10 atomes de carbone ou un radical cycloalkoxy ayant jusqu'à 6 atomes de carbone ou un radical alkényloxy ou alkynyloxy linéaire ou ramifié ayant 3 à 6 atomes de carbone.

b) un halogène, un radical cycloalkyle ayant 3 à 7 atomes de carbone, un radical phényle, α - ou β -thiényle ou α- ou β -furyle non substitué, qui, de leur côté, sont substitués sur le noyau 1 à 3 fois par un halogène, un radical trifluorométhyle et/ou un radical alkyle ou alkoxy ayant 1 à 4 atomes de carbone,

c) un radical phénoxy, α - ou β -thiényloxy ou cycloalkoxy ayant 3 à 7 atomes de carbone non substitué ou un des radicaux cités, qui, de son côté, est substitué sur le noyau 1 à 3 fois par un halogène, un radical trifluorométhyle et/ou un radical alkyle ou alkoxy ayant chacun 1 à 4 atomes de carbone,

d) un radical 1-imidazolyle,

caractérisé en ce que

a) on fait réagir les 1-imidaxolylméthylbenzaldéhydes ortho-, méta- et parasubstitués correspondants de formule II

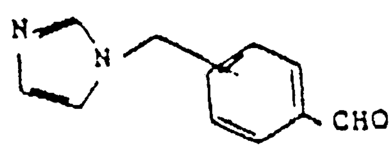

II

par la méthode, de Horner-Emmons-Wittig avec un ester phosphonique de formule générale III,

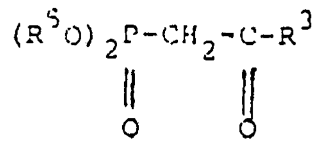

III

$R^3$ ayant la signification indiquée dans la formule générale I et $R^5$ représentant un radical alkyle en $C_1$ - $C_4$, pour obtenir des composés de formule générale IV,

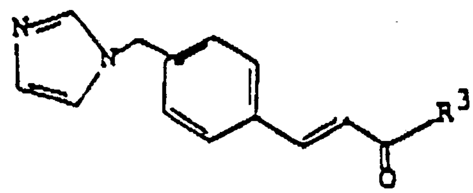

IV

$R^3$ ayant la signification indiquée pour la formule I,

b) éventuellement on réduit les énones de formule générale IV par un réducteur pour obtenir les alcools de formule générale V,

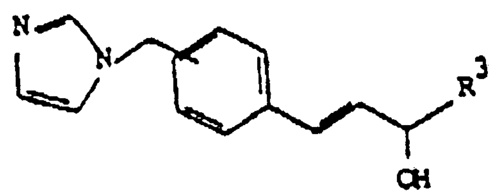

V

$R^3$ ayant la signification indiquée par la formule I,

c) éventuellement on fait réagir les alcools de formule V, sous la forme de leurs racémates ou sous la forme d'énantiomères purs, avec un dérivé réactif d'un acide carboxylique pour obtenir les esters de formule générale VI

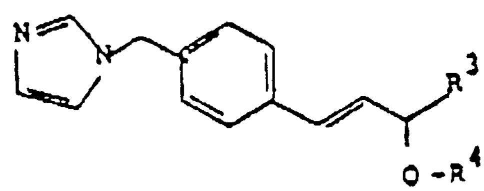

VI

dans laquelle $R^3$ a la signification indiquée pour la formule I et $R^4$ représente un radical acyle aliphatique ramifie ou non ramifié ayant jusqu'à 10 atomes de carbone ou le radical benzyl carbonyle ou benzoyle, le noyau phényle pouvant être substitué 1 à 3 fois par un halogène ou un radical alkyle en $C_1$-$C_4$, ou le radical

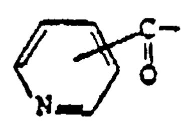

d) éventuellement on fait réagir les alcools de formule V, sous la forme de leurs racémates ou sous la forme d'énantiomères purs, avec un halogénure, un tosylate ou un mésylate correspondant pour obtenir les éthers de formule générale VII,

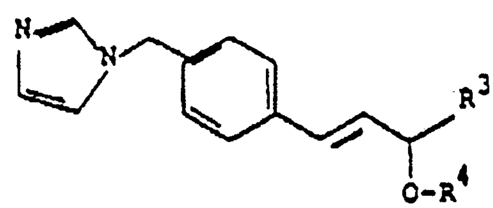

VII

dans laquelle $R^4$ représente un radical alkyle en $C_1$-$C_{10}$, un radical benzyle, dont le noyau phényle peut être substitué 1 à 3 fois par un halogène ou un radical alkyle en $C_1$-$C_4$, ou le radical

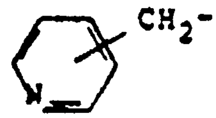

2. Procédé selon la revendication 1 caractérisé en ce que dans la formule I,

$R^1$ et $R^2$ représentent, avec l'atome de carbone qui les porte, le groupe carbonyle, ou $R^1$ représente l'hydrogène et $R^2$ le radical $-OR^4$, $R^4$ représentant l'hydrogène ou

a) un radical alkanoyle ramifié ou non ramifié ayant jusqu'à 6 atomes de carbone,

b) le radical benzylcarbonyle ou benzoyle, dont le noyau phényle est non substitué ou substitué 1 fois par le fluor, le chlore ou un radical méthyle,

c) le radical

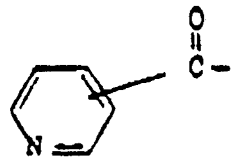

d) un radical alkyle ramifié ou non ramifié ayant 1 à 6 atomes de carbone,

e) le radical benzyle, dont le noyau phényle est non substitué ou substitué 1 fois par le chlore, le fluor ou un radical méthyle, ou

f) le radical

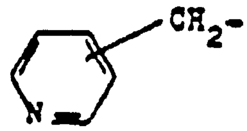

$R^3$ représente un radical cycloaliphatique ayant 3 à 8 atomes de carbone, un radical alkyle linéaire ou ramifié ayant jusqu'à 8 atomes de carbone, un radical hydrocarboné aliphatique insaturé linéaire ou ramifié ayant 3 a 8 atomes de carbone, les radicaux aliphatiques pouvant de leur côté être substitués par:

a) un radical alkoxy linéaire ou ramifié ayant jusqu'à 6 atomes de carbone ou un radical cycloalkoxy ayant jusqu'à 6 atomes de carbone ou un radical alkényloxy ou alkynyloxy linéaire ou ramifié ayant 3 à 6 atomes de carbone,

b) un radical cycloalkyle ayant 3 à 7 atomes de carbone, un radical phényle, α- ou β -thiényle ou α- ou β -furyle non substitué,

c) un radical phénoxy, α - ou β -thiényloxy ou cycloalkoxy ayant 3 à 7 atomes de carbone ou un des radicaux cités, qui de son côté est substitué sur le noyau 1 à 3 fois par un halogène, un radical trifluorométhyle et/ou un radical alkyle ou alkoxy ayant chacun 1 à 4 atomes de carbone ou

d) un radical 1-imidazolyle.

3. Procédé selon la revendication 1 caractérisé en ce que la formule I,

$R^1$ et $R^2$ représentent, avec l'atome de carbone qui les porte, le groupe carbonyle, ou $R^1$ représente l'hydrogène et $R^2$ le radical $-OR^4$, $R^4$ représentant l'hydrogène, un radical benzyle, un radical alkyle ayant 1 à 6 atomes de carbone ou le radical

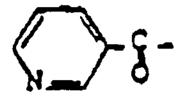

ou

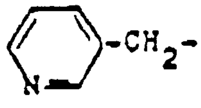

$R^3$ représente un radical cycloaliphatique ayant 3 à 8 atomes de carbone, un radical alkyle linéaire ou ramifié ayant jusqu'à 8 atomes de carbone, un radical hydrocarboné aliphatique insaturé ou linéaire ou ramifié ayant 3 à 8 atomes de carbone, les radicaux aliphatiques pouvant de leur côté être substitués par:

a) un radical alkoxy linéaire ou ramifié ayant jusqu'à 10 atomes de carbone,
b) un radical cycloalkyle ayant 3 à 7 atomes de carbone, un radical phényle, α- ou β-thiényle ou α- ou β-furyle non substitué,
c) un radical phénoxy, α- ou β-thiényloxy ou cycloalkoxy ayant 3 à 7 atomes de carbone non substitué ou
d) un radical 1-imidazolyle.